# EUROPEAN PATENT APPLICATION

(11) **EP 1 593 739 A1**
(43) Date of publication of application: **09.11.2005**
(21) Application number: 04711026.7
(22) Date of filing: 13.02.2004
(51) Int. Cl.: C12N 15/00, C12P 7/40, C12N 9/02

(54) **PROCESS FOR BIOCHEMICAL PRODUCTION OF GLYOXYLIC ACID**

(30) Priority: 14.02.2003 JP 2003036303; 26.09.2003 JP 2003336234
(71) Applicant: KANEKA CORPORATION, Osaka-shi, Osaka 530-8288 (JP)
(72) Inventor: IWASAKI, Akira, Kakogawa-shi, Hyogo 675-0068 (JP); MATSUMOTO, Takehiko, Takasago-shi, Hyogo 676-0807 (JP); WASHIDA, Motohisa, Kobe-shi, Hyogo 655-0872 (JP); WATANABE, Hiroshi, Kobe-shi, Hyogo 651-2276 (JP); HASEGAWA, Junzou, Akashi-shi, Hyogo 674-0057 (JP); SHIMIZU, Sakayu, Kyoto-shi, Kyoto 616-8212 (JP)
(74) Representative: HOFFMANN EITLE
(86) International application number: PCT/JP2004/001577
(87) International publication number: WO 2004/072281

(57) **Abstract**

The present invention provides an industrially advantageous process for biochemical production of glyoxylic acid from glyoxal. More specifically, the present invention provides a process for production of glyoxylic acid, which is **characterized in that** the process comprises allowing oxidoreductase that can convert glyoxal into glyoxylic acid, such as oxidase and dehydrogenase, to act on glyoxal, so as to convert glyoxal into glyoxylic acid.

SEQUENCE LISTING

<110> Kaneka Corporation

<120> Biochemical process for producing glyoxylic acid

<130> FP-8610PCT

<150> JP 2003-36303
<151> 2003-02-14

<150> JP 2003-336234
<151> 2003-09-26

<160> 42

<210> 1
<211> 189
<212> PRT
<213> Streptomyces sp. KNK269

<223> amino acid sequence of subunit gamma of aldehyde oxidase

<400> 1
Gly Ala Asp Leu Glu Pro Glu Pro Val Val Asp Glu His Ser Thr Val
1               5                   10                  15
Thr Leu Asn Val Asn Gly Asp Pro Thr Thr Leu Thr Val Asp His Arg
            20                  25                  30

Thr Thr Val Leu Glu Thr Leu Arg Glu Ser Phe Gly Leu Thr Gly Ala
        35                  40                  45
Lys Lys Gly Cys Asp His Gly Gln Cys Gly Ala Cys Thr Val Leu Val
    50                  55                  60
Asp Gly Arg Arg Val Asn Ser Cys Leu Leu Leu Ala Val Ala Gln Asp
65                  70                  75                  80
Gly Ala Thr Ile Thr Thr Val Glu Gly Leu Ala Asp Gly Glu Asp Leu
                85                  90                  95
His Pro Val Gln Arg Ala Phe Val Glu Arg Asp Ala Leu Gln Cys Gly
            100                 105                 110
Phe Cys Thr Pro Gly Gln Ile Cys Ser Ala Val Gly Met Leu Arg Glu
        115                 120                 125
Ala Ala Asp Gly His Pro Ser His Val Thr Gly Pro Asp Thr Ala Ala
    130                 135                 140
Gly Pro Asp Ala Ala Thr Gly Pro Gly Gly Pro Val Ala Leu Asp Ala
145                 150                 155                 160
Asp Glu Ile Arg Glu Arg Met Ser Gly Asn Ile Phe Arg Cys Gly Ala
                165                 170                 175
Tyr His Arg Ile Val Glu Ala Ile Glu Asp Val Ile Glu
            180                 185

<210> 2
<211> 333
<212> PRT
<213> Streptomyces sp. KNK269

<223> amino acid sequence of subunit beta of aldehyde oxidase

<400> 2

Met Lys Pro Phe Gly Tyr Val Arg Pro Ala Ser Pro Asp Glu Ala Val
1               5                   10                  15

Arg Leu Cys Ala Glu Gly Ser Gly Ala Arg Phe Leu Gly Gly Gly Thr
            20                  25                  30

Asn Leu Val Asp Leu Met Lys Leu Gly Val Glu Ala Pro Arg Val Leu
        35                  40                  45

Ile Asp Val Ala Gly Leu Pro Leu Asp Arg Val Thr Arg Thr Ala Asp
    50                  55                  60

Gly Gly Leu Ala Ile Gly Ala Thr Val Arg Asn Ser Asp Leu Ala Ala
65                  70                  75                  80

His Pro Asp Val Thr Asp Arg Tyr Pro Val Leu Ser Gln Ala Leu Leu
                85                  90                  95

Ala Gly Ala Ser Gly Gln Leu Arg Asn Ser Ala Thr Thr Gly Gly Asn
            100                 105                 110

Leu Leu Gln Arg Thr Arg Cys Arg Tyr Phe Gln Asp Val Ser Lys Pro
        115                 120                 125

Cys Asn Lys Arg Leu Pro Gly Ser Gly Cys Pro Ala Arg Asp Gly Thr
    130                 135                 140

His Arg Asp Leu Ala Ile Leu Gly His Ser Pro Glu Cys Val Ala Thr
145                 150                 155                 160

Asn Pro Ser Asp Met Ala Val Ala Leu Ala Ala Leu Asp Ala Thr Val
                165                 170                 175

Val Leu Leu Gly Pro Glu Gly Glu Arg Ala Val Pro Leu Thr Glu Phe
            180                 185                 190

His Arg Leu Pro Gly Glu Asn Pro Asp Gln Asp Thr Ala Ile Arg Thr
        195                 200                 205

Gly Glu Leu Ile Thr Glu Val Val Leu Pro Pro Pro Ala Pro Gly Ala
    210                 215                 220
Ala Thr Arg Tyr Arg Lys Ala Arg Asp Arg Ala Ser Phe Ala Phe Ala
225                 230                 235                 240
Leu Val Ser Val Ala Ala Thr Leu Arg Val Asp Ala Gly Arg Val Glu
                245                 250                 255
His Ala Thr Leu Ala Phe Gly Gly Val Ala His Arg Pro Trp Arg Ala
            260                 265                 270
Arg Arg Ala Glu Ala Glu Leu Arg Gly Ala Pro Ala Thr Pro Ala Val
        275                 280                 285
Phe Gln His Ala Leu Ile Ala Glu Leu Ala Glu Ala Arg Pro Leu Arg
    290                 295                 300
Asp Asn Val Phe Lys Val Asp Leu Ala Arg Arg Leu Ala Leu Asp Val
305                 310                 315                 320
Leu Gly Glu Leu Thr Glu Arg Gln Pro Thr Arg Thr Gly
                325                 330

<210> 3
<211> 693
<212> PRT
<213> Streptomyces sp. KNK269

<223> amino acid sequence of subunit alpha of aldehyde oxidase

<400> 3
Met Thr Thr Arg Ala Pro Gln Phe Pro Gly Ala Pro Val Val Arg Arg
1               5                   10                  15

Glu Ala Arg Asp Lys Val Thr Gly Thr Ala Arg Tyr Ala Ala Asp Arg
            20                  25                  30
Thr Pro Ala Gly Cys Leu Tyr Ala Trp Thr Val Pro Ala Ala Val Ala
        35                  40                  45
Ala Gly Arg Val Thr Ala Val Arg Ala Val Asp Ala Leu Ala Val Pro
    50                  55                  60
Gly Val His Thr Val Leu Thr His Asp Asn Ala Pro Glu Leu Arg Glu
65                  70                  75                  80
Pro Asp Asp Pro Ile Leu Ala Val Leu Gln Asp Asp Arg Val Pro His
                85                  90                  95
His Gly Trp Pro Ile Ala Leu Val Ile Ala Glu Ser Pro Glu Ala Ala
            100                 105                 110
Arg Thr Gly Ala Gly Glu Leu His Val Glu Tyr Ala Arg Asp Glu His
        115                 120                 125
Asp Val Thr Leu Thr Glu Asp His Pro Gly Leu Tyr Thr Pro Glu Glu
    130                 135                 140
Ala Asn Gly Gly Glu Pro Ala Val Arg Val Arg Gly Asp Val Glu Arg
145                 150                 155                 160
Ala Leu Ala Ala Ser Pro Val Gln Val Asp Ala Thr Tyr Arg Met Val
                165                 170                 175
Ala Leu His Asn His Pro Met Glu Pro His Ala Ala Thr Ala Val Arg
            180                 185                 190
Ala Asp Gly His Leu Thr Val His Asp Ser Ser Gln Gly Ser Thr Lys
        195                 200                 205
Val Arg Glu Ser Leu Ala Gln Ala Phe Gly Leu Ala Ala Asp Arg Ile
    210                 215                 220
Thr Val Val Ser Glu His Val Gly Gly Gly Phe Gly Ser Lys Gly Thr
225                 230                 235                 240

Thr Arg Pro Gln Gly Val Leu Ala Ala Met Ala Ala Gln His Thr Gly
                245                 250                 255
Arg Pro Val Lys Leu Val Phe Pro Arg Ala Gln Leu Ala Glu Val Val
            260                 265                 270
Gly His Arg Ala Pro Thr Ile Gln Arg Val Arg Leu Gly Ala Asp Leu
        275                 280                 285
Asp Gly Val Leu Thr Ala Val Ser His Glu Val Val Thr Gln Thr Ser
    290                 295                 300
Thr Val Lys Glu Phe Val Glu Gln Ala Ala Val Pro Ala Arg Val Met
305                 310                 315                 320
Tyr Ala Ser Pro His Ser Arg Thr Ala His Arg Val Thr Ala Leu Asp
                325                 330                 335
Val Pro Ser Pro Ser Trp Met Arg Ala Pro Gly Glu Ala Ser Gly Met
            340                 345                 350
Tyr Ala Leu Glu Ser Ala Met Asp Val Leu Asp Ser Ala Leu Gly Leu
        355                 360                 365
Asp Pro Val Glu Leu Arg Leu Arg Asn Glu Pro Glu Thr Glu Pro Asp
    370                 375                 380
Ser Gly Arg Pro Phe Ser Ser Arg Gly Leu Ala Ala Cys Leu Arg Asp
385                 390                 395                 400
Gly Ala Ala Arg Phe Gly Trp Gln Asp Arg Asp Pro Arg Pro Ala Ala
                405                 410                 415
Arg Gln Glu Gly Arg Trp Leu Ile Gly Thr Gly Val Ala Ser Ala Thr
            420                 425                 430
Tyr Pro Val Leu Leu Ser Arg Ser Thr Ala Ser Ala His Ala Thr Arg
        435                 440                 445
Asp Gly Asp Leu Ser Ile Ala Val Asn Ala Thr Asp Ile Gly Thr Gly
    450                 455                 460

Ala Arg Thr Val Leu Ala Gln Ile Ala Ala Asp Val Leu Gly Val Ala
465                 470                 475                 480
Pro Glu Ala Leu Arg Val Asp Ile Gly Ser Thr Asp Leu Pro Ala Ala
                485                 490                 495
Pro Leu Ala Gly Gly Ser Ser Gly Thr Ala Ser Trp Gly Trp Ser Val
            500                 505                 510
His Lys Ala Ala Thr Ala Leu Ala Ala Arg Leu Ala Glu His Arg Gly
        515                 520                 525
Pro Leu Pro Ala Asp Gly Ile Thr Thr Thr Ala Asp Thr Gly Gln Glu
    530                 535                 540
Thr Gly Glu Glu Ser Pro Tyr Ala Arg His Ala Phe Gly Ala His Phe
545                 550                 555                 560
Ala Glu Thr Ala Val Asp Ala Val Thr Gly Glu Val Arg Val Arg Arg
                565                 570                 575
Leu Leu Gly Val Tyr Ala Ala Gly Arg Ile Leu Asn Glu Arg Thr Ala
            580                 585                 590
Arg Ser Gln Phe Thr Gly Gly Met Val Met Gly Ile Gly Met Ala Leu
        595                 600                 605
Thr Glu Gly Cys Gly Ile Asp Pro Gly Phe Gly Asp Phe Thr Ala Lys
    610                 615                 620
Asp Leu Ala Ser Tyr His Val Pro Val Cys Ala Asp Thr Ala Asp Ile
625                 630                 635                 640
Gln Ala His Trp Ile Glu Glu Asp Asp Arg His Leu Asn Pro Met Gly
                645                 650                 655
Ser Lys Gly Ile Gly Glu Ile Gly Ile Val Gly Thr Ala Ala Ala Ile
            660                 665                 670
Gly Asn Ala Val Arg His Ala Thr Gly Ala Arg Leu Arg Glu Leu Pro
        675                 680                 685

Leu Thr Thr Asp Thr
    690

<210> 4
<211> 536
<212> DNA
<213> Streptomyces sp. KNK269

<223> DNA sequence of subunit gamma of aldehyde oxidase

<400> 4
gaa cac tcc acc gtg acc ctg aac gtc aac ggc gac ccg acg acg ctg 48
acc gtc gac cac cgc acc acg gtc ctg gag acg ctg cgc gag agc ttc 96
ggg ctc acc ggg gcc aag aag ggc tgc gac cac ggc cag tgc ggg gcc 144
tgc acg gtc ctc gtc gac ggg cgg cgg gtc aac agc tgc ctg ctg ctc 192
gcc gtc gcc cag gac ggc gcc acg atc acc acc gtg gaa ggg ctc gcc 240
gac ggc gag gac ctg cac ccg gtg cag cgg gcg ttc gtc gag cgc gac 288
gcc ctc cag tgc ggc ttc tgc acc ccg ggc cag atc tgc tcg gcc gtg 336
ggc atg ctc cgc gag gcc gcg gac ggc cac ccc tcg cac gtc acc gga 384
ccg gac acc gcc gcc gga ccg gac gcc gct acc gga ccg ggc ggg ccc 432
gtc gca ctc gac gcg gac gag atc cgg gaa cgg atg agc ggc aac atc 480
ttc cgg tgc ggg gcc tat cat cgg atc gta gaa gcg atc gag gac gtg 528
atc gag tga 536

<210> 5
<211> 1002

<212> DNA
<213> Streptomyces sp. KNK269

<223> DNA sequence of subunit beta of aldehyde oxidase

<400> 5
gtg aaa ccg ttc ggc tat gtg cgc ccc gcc tcc ccc gac gag gct gtc 48
cgg ctc tgc gcg gaa ggc tcc ggc gcc cgc ttc ctg ggc ggc ggc acc 96
aat ctg gtg gac ctg atg aag ctc ggc gtc gag gcg ccc cgg gtc ctc 144
atc gac gtc gca ggg ctt ccg ctg gac cgg gtg acc cgg acg gcc gac 192
ggc ggc ctc gcc atc ggc gcc acc gtg cgc aac agc gac ctc gcc gcc 240
cac ccc gac gtg acg gac cgg tac ccc gtg ctg tcc cag gcg ctg ctg 288
gcc ggg gcc tcc ggt cag ctc cgc aac tcc gcc acc acc ggc ggc aac 336
ctg ctc cag cgc acc cgg tgc cgc tac ttc cag gac gtc tcc aag ccc 384
tgc aac aaa cgg ctt ccc ggc tcc ggc tgc ccc gca cgc gac ggc act 432
cac cgc gac ctc gcg atc ctc ggg cac tcg ccg gag tgc gtg gcc acc 480
aac ccg tcc gac atg gcc gtg gcc ctc gcc gcg ctc gac gcg acc gtg 528
gtg ctg ctg ggg ccc gag ggc gaa cgg gcc gtc ccg ctc acc gag ttc 576
cac cgg ttg ccg ggg gag aac ccc gac cag gac acc gcg atc agg acc 624
ggc gag ctg atc acc gag gtg gtg ctg ccg ccg ccg gcg ccc ggg gcg 672
gcc acc cgc tac cgc aag gcc cgc gac cgc gcc agc ttc gcc ttc gcg 720
ctc gtc tcc gtc gcc gcc acg ctc cgg gtc gac gcc ggc cgc gtc gag 768
cac gcc acc ctc gcg ttc ggc ggc gtc gcc cac cgg ccg tgg cgg gcc 816
cgc agg gcc gag gcc gag ctg cgc ggc gcc ccg gcc acg ccc gcc gtg 864
ttc cag cac gcc ctg atc gcc gaa ctg gcc gag gcg cga ccg ctg cgc 912
gac aac gtc ttc aag gtg gac ctc gcc cgc cgg ctc gcc ctc gac gtg 960
ctc ggc gaa ctc acc gag cgg caa ccc acc cgg acc ggc tga 1002

<210> 6

<211> 2055

<212> DNA

<213> Streptomyces sp. KNK269

<223> DNA sequence of subunit alpha of aldehyde oxidase

<400> 6

atg acc acc cga gcc ccg cag ttc ccc ggt gcg ccc gtc gtc cgc aga 48

gag gcc cgg gac aag gtc acc ggc acc gcc cgc tac gcc gcc gac cgc 96

acg ccc gcc ggc tgc ctc tac gcc tgg acc gtc ccg gcc gcc gtc gcc 144

gcc gga cgc gtc acc gcc gta cgg gcc gtc gac gct ctc gcc gtc ccc 192

ggc gtc cac acc gtc ctc acc cac gac aac gcg ccc gaa ctc cgg gag 240

ccc gac gac ccg atc ctc gcc gtg ctc cag gac gac cgg gtc ccg cac 288

cac ggc tgg ccc atc gcc ctc gtg atc gcg gag tcc ccg gag gcc gcc 336

cgg aca ggg gcc ggg gag ctc cac gtc gaa tac gca cgc gac gag cac 384

gac gtg acc ctc acc gaa gac cat ccg ggt ctc tac acg ccc gag gag 432

gcc aac ggc ggt gag ccc gcc gtc cgg gtg cgc ggc gac gtg gaa cgg 480

gcc ctg gcg gcc tcg ccc gtc cag gtc gac gcc act tac cgg atg gtc 528

gcg ctg cac aac cac ccc atg gaa ccg cac gcc gcc acc gcg gtg cgg 576

gcc gac ggg cac ctc acc gtc cac gac tcc agc cag ggc tcc acc aag 624

gtc cgc gag agt ctg gcc cag gcg ttc gga ctg gcg gcg gac cgg atc 672

acc gtg gtc tcc gag cac gtc ggc ggc ggc ttc ggc tcg aag ggc acc 720

acc cgc ccc cag gga gtc ctg gcg gcg atg gcc gcc cag cac acc ggg 768

cgg ccg gtg aag ctg gtg ttc ccc cgc gcc cag ctg gcc gag gtc gtc 816

ggc cac cgc gcc ccc acc atc cag cga gtc agg ctc ggc gcg gac ctg 864

gac ggg gtg ctc acc gcg gtc agc cac gag gtc gtc acc cag acc tcc 912

acc gtg aag gag ttc gtc gag cag gcc gcc gtg ccc gcc cgc gtc atg 960
tac gcc tcg ccg cac agc cgg acc gcg cac cgg gtg acc gcc ctc gac 1008
gtc ccc agc ccc tcc tgg atg cgc gcc ccg ggg gag gcc tcc ggg atg 1056
tac gcc ctg gag tcc gcc atg gac gtg ctg gac tcc gca ctc ggc ctg 1104
gac ccc gtc gag ctg cgg ctc cgc aac gaa ccg gag acc gaa ccg gac 1152
agc ggc cgc ccc ttc agc agc cgg ggc ctc gcc gcc tgt ctg cgc gac 1200
ggc gcg gcc cgg ttc ggc tgg cag gac cgc gac ccc cgg ccc gca gcc 1248
cgc cag gaa ggc cgc tgg ctc atc ggt acg ggc gtg gcc tcg gcg acc 1296
tac ccc gtc ctc ctc tcc cgg tcc acc gcg agc gcc cac gcc acc cgg 1344
gac ggt gac ctg agc atc gcc gtc aac gcc acc gac atc ggc acc ggg 1392
gcc cgc acc gtc ctg gcc cag atc gcc gcc gac gtg ctc ggc gtc gcc 1440
ccg gag gcc ctg cgc gtc gac atc ggc tcc acc gac ctg ccc gcc gcc 1488
ccg ctg gcc gga ggc tcc tcc ggc acc gcc tcc tgg ggc tgg tcc gtg 1536
cac aag gcg gcg acc gcc ctg gcc gcc cgc ctc gcg gag cac cgg ggc 1584
ccc ctg ccc gcc gac ggg atc acc acc acc gcc gac acc gga cag gag 1632
acc ggc gag gag tcc ccg tac gcc cgg cac gcc ttc ggc gcg cac ttc 1680
gcc gag acg gcc gtc gac gcc gtc acg ggc gag gtg cgc gta cgc cgc 1728
ctc ctc ggc gtg tac gcc gcc ggg cgg atc ctc aac gag cgc acc gca 1776
cgc tcc cag ttc acc ggt ggc atg gtg atg ggc atc ggc atg gcc ctc 1824
acc gag ggc tgc ggc atc gac ccg ggc ttc ggc gac ttc acc gcc aag 1872
gac ctc gcc tcc tac cac gtg ccc gtc tgc gcc gac acg gcc gac atc 1920
cag gcg cac tgg atc gag gag gac gac cgc cac ctc aac ccg atg ggc 1968
agc aag ggc atc ggc gag atc ggg atc gtc ggc acc gcc gcc gcc att 2016
ggc aac gcg gtc cgc cac gcc acc ggc gcc cgg ctg cgc 2055

<210> 7
<211> 1115

<212> PRT
<213> Microbacterium sp. KNK011

<223> amino acid sequence of aldehyde oxidase containing signal peptide

<400> 7
Met Pro Val Pro Ala Val Leu Ser Gly Pro Arg Arg Arg Ser Thr Arg
1               5                   10                  15
Val Ala Arg Thr Leu Ala Ala Gly Leu Leu Gly Gly Ala Leu Val Ala
            20                  25                  30
Thr Gly Ala Ile Leu Pro Ala Gly Ala Ala Val Ala Pro Ala Val Asn
        35                  40                  45
Thr Pro Ala Asp Leu Pro Lys Gln Glu Pro Gly Val Thr Leu Arg Thr
    50                  55                  60
Tyr Ser Thr Pro Pro Leu Thr Glu Leu Cys Thr Leu Lys Ser Gly Gln
65                  70                  75                  80
Thr Pro Asn Val Asp Lys Leu Met Ser Thr Ile Asp Trp Arg Thr Asp
                85                  90                  95
Glu Gln Phe Gly Ala Gly Asp Asn Phe Ile Thr His Ala Leu Ala Asn
            100                 105                 110
Leu Thr Val Ser Thr Pro Gly Gln Tyr Ala Phe Arg Leu Thr Ser Asp
        115                 120                 125
Asp Gly Ser Arg Leu Thr Leu Asp Gly Thr Gln Leu Ile Asp Asn Asp
    130                 135                 140
Gly Leu His Gly Ala Glu Ser Val Glu Gly Thr Val Thr Leu Asp Val
145                 150                 155                 160
Gly Val His Asp Leu Phe Val Glu Met Phe Glu Ala Thr Asn Gly Gln
                165                 170                 175

Gln Leu Thr Val Glu Trp Lys Val Pro Gly Ser Ser Ser Phe Thr Val
            180                 185                 190
Ile Pro Asn Ser Val Leu Ser Thr Glu Ala Gly Val Val Arg Val Thr
        195                 200                 205
Ala Pro Gly Thr Lys Tyr Cys Glu Gly Ser Thr Asp Ser Ala Gly Asp
    210                 215                 220
Gly Leu Arg Leu Asp Ala Val Asn Pro Asn Tyr Thr Leu Val Asn Leu
225                 230                 235                 240
Arg Pro Glu Gly Phe Thr Pro Lys Val Ser Gly Leu Ala Phe Leu Gly
                245                 250                 255
Asn Gly Asp Leu Ala Val Leu Thr Thr Gly Ser Val Asn Ser Gly Gly
            260                 265                 270
Trp Asp Thr Ser Thr Pro Gly Lys Val Phe Val Leu Lys Gly Ala Gln
        275                 280                 285
Ala Ala Asp Gly Pro Glu Asp Val Thr Val Val Glu Ala Ala Gly Gly
    290                 295                 300
Leu Leu Asn Pro Met Gly Ile Asp Val Ile Asp Asp Lys Ile Tyr Val
305                 310                 315                 320
Ser Glu Arg Tyr Gln Leu Thr Glu Leu Ser Asp Thr Asn Gly Asp Gly
                325                 330                 335
Ser Tyr Glu Thr Lys Arg Lys Val Ala Glu Tyr Pro Ser Gly Asn Asn
            340                 345                 350
Phe His Glu Phe Ala Phe Gly Leu Ile His Asp Glu Lys Asn Phe Tyr
        355                 360                 365
Val Asn Leu Ser Val Ala Ile Asp Asn Gly Gly Ala Thr Thr Asn Pro
    370                 375                 380
Gln Pro Ala Lys Asn Arg Gly Thr Ser Val Lys Ile Asp Arg Ala Thr
385                 390                 395                 400

Gly Ala Ile Ser Tyr Val Ala Gly Gly Leu Arg Thr Pro Asn Gly Val
                405                 410                 415
Ala Phe Gly Pro Glu Asn Glu Leu Phe Ala Met Asp Asn Gln Gly Ala
            420                 425                 430
Trp Leu Pro Ala Asn Lys Leu Val Asn Val Lys Gln Asp Arg Phe Phe
        435                 440                 445
Asn His Tyr Thr Asn Pro Ala Gly Pro Phe Asp Gln Asn Pro Val Thr
    450                 455                 460
Ala Pro Val Val Trp Ile Pro Gln Asn Glu Ile Gly Asn Ser Pro Ser
465                 470                 475                 480
Thr Pro Ile Met Leu Lys Asp Gly Pro Phe Ala Gly Gln Met Met Phe
                485                 490                 495
Gly Asp Val Thr Tyr Gly Gly Leu Gln Arg Ala Phe Leu Glu Lys Val
            500                 505                 510
Asp Gly Glu Phe Gln Gly Ala Val Phe Arg His Ser Ala Gly Phe Glu
        515                 520                 525
Val Gly Val Asn Arg Val Ile Glu Gly Pro Asp Thr Ser Leu Tyr Ile
    530                 535                 540
Gly Gly Thr Gly Glu Gly Gly Asn Trp Gly Glu Ala Gly Lys Leu Thr
545                 550                 555                 560
Tyr Gly Leu Gln Lys Leu Val Pro Asn Thr Thr Gln Asn Ala Phe Asp
                565                 570                 575
Met Lys Ser Met Lys Val Val Glu Gly Gly Phe Glu Ile Glu Tyr Thr
            580                 585                 590
Gln Pro Leu Ser Asp Glu Thr Leu Ala Asn Leu Ala Ser Ala Tyr Arg
        595                 600                 605
Ala Glu Gln Trp Arg Tyr Leu Pro Thr Ser Thr Tyr Gly Gly Pro Lys
    610                 615                 620

Val Asp Glu Glu Ile Leu Ser Leu Thr Gly Ala Thr Ala Ser Ala Asp
625                 630                 635                 640
Arg Lys Thr Val Thr Val Ser Phe Asp Gly Leu Lys Gln Gly Arg Val
                645                 650                 655
Val His Leu Arg Ser Pro Gln Pro Phe Ala Ala Ala Ser Gly Asp Thr
            660                 665                 670
Leu Trp Ser Thr Glu Ala Trp Tyr Thr Leu Asn Ser Leu Pro Gly Tyr
        675                 680                 685
Val Ser Pro Ala Asp Gln Gly Trp Tyr Glu Ala Glu Glu Ala Arg Leu
    690                 695                 700
Ile Gly Gly Ala Lys Phe Asp Ala Glu His Ser Gly Tyr Ser Gly Ala
705                 710                 715                 720
Gly Phe Ala Gly Gly Met Trp Gln Ala Gly Ser Ala Phe Glu Phe Thr
                725                 730                 735
Val Asn Ala Glu Thr Ala Gly Thr Val Pro Val Ser Val Arg Tyr Ser
            740                 745                 750
Asn Gly Pro Asn Pro Ala Pro Gly Ser Lys Asp Val Asn Leu Tyr Val
        755                 760                 765
Asn Gly Gln Asn Leu Gly Lys Trp Asp Phe Ala Ser Thr Gly Asp Trp
    770                 775                 780
Lys Thr Trp Ala Thr Ile Thr Arg Asp Met Pro Leu Val Ala Gly Thr
785                 790                 795                 800
Asn Thr Ile Ala Leu Lys Tyr Glu Thr Gly Asn Lys Gly Asn Ile Asn
                805                 810                 815
Val Asp Val Leu Ser Ile Gly Thr Ala Asp Ile Cys Ala Pro Ser Gln
            820                 825                 830
Val Glu Asp Gly Tyr Arg Pro Leu Phe Asp Gly Thr Leu Glu Ser Leu
        835                 840                 845

Asn Ala Gly Trp Arg Met Ala Gly Pro Gly Gly Phe Gly Arg Gln Asn
    850                 855                 860
Asp Cys Ser Ile Arg Gly Glu Gly Gly Met Gly Leu Leu Trp His Lys
865                 870                 875                 880
Ala Gln Glu Leu Asn Glu Tyr Ser Leu Lys Leu Asp Trp Lys Leu Ile
                885                 890                 895
Ala Asp His Asn Gly Gly Val Phe Val Gly Phe Pro Asp Pro Lys Asn
            900                 905                 910
Asp Pro Trp Ile Ala Val Asn Gln Gly Tyr Glu Ile Gln Ile Asp Ala
        915                 920                 925
Ser Asp Ala Ala Asp Arg Thr Thr Gly Ala Ile Tyr Thr Phe Gln Gly
    930                 935                 940
Ala Asp Ala Asp Ala Val Lys Ala Ser Leu Lys Pro Val Gly Gln Trp
945                 950                 955                 960
Asn Ala Tyr Glu Ile Val Val Lys Gly Gln Thr Ile Lys Ile Phe Leu
                965                 970                 975
Asn Gly Thr Leu Val Asn Asp Phe Thr Ser Thr Asp Pro Ala Arg Asp
            980                 985                 990
Leu Ser Gln Gly Phe Ile Gly Leu Gln Asn His Gly Gly Gly Glu Ala
        995                 1000                1005
Val Ser Tyr Arg Asn Val Arg Val Lys Asp Ile Asp Glu Pro Ala Pro
    1010                1015                1020
Leu Ala Val Thr Ala Ser Ala Glu Val Lys Cys Leu Ala Lys Lys Ala
1025                1030                1035                1040
Ser Val Thr Ala Arg Ala Thr Asn Thr Asp Thr Val Pro Val Asp Val
                1045                1050                1055
Thr Leu Thr Thr Ala Trp Gly Glu Arg Val Ile Pro Ala Val Gln Pro
            1060                1065                1070

Gly Ala Thr Val Phe His Thr Phe Thr Thr Arg Ala Ala Ser Val Pro
        1075                1080                1085
Ala Gly Glu Ala Thr Val Thr Ala Thr Gly Asp Gly Arg Thr Gly Gly
    1090                1095                1100
Ala Thr Ala Ser Tyr Ala Ala Lys Ser Cys Gly
1105                1110                1115

<210> 8
<211> 1069
<212> PRT
<213> Microbacterium sp. KNK011

<223> amino acid sequence of aldehyde oxidase

<400> 8
Val Asn Thr Pro Ala Asp Leu Pro Lys Gln Glu Pro Gly Val Thr Leu
1               5                   10                  15
Arg Thr Tyr Ser Thr Pro Pro Leu Thr Glu Leu Cys Thr Leu Lys Ser
            20                  25                  30
Gly Gln Thr Pro Asn Val Asp Lys Leu Met Ser Thr Ile Asp Trp Arg
        35                  40                  45
Thr Asp Glu Gln Phe Gly Ala Gly Asp Asn Phe Ile Thr His Ala Leu
    50                  55                  60
Ala Asn Leu Thr Val Ser Thr Pro Gly Gln Tyr Ala Phe Arg Leu Thr
65                  70                  75                  80
Ser Asp Asp Gly Ser Arg Leu Thr Leu Asp Gly Thr Gln Leu Ile Asp
                85                  90                  95

Asn Asp Gly Leu His Gly Ala Glu Ser Val Glu Gly Thr Val Thr Leu
            100                 105                 110
Asp Val Gly Val His Asp Leu Phe Val Glu Met Phe Glu Ala Thr Asn
        115                 120                 125
Gly Gln Gln Leu Thr Val Glu Trp Lys Val Pro Gly Ser Ser Ser Phe
    130                 135                 140
Thr Val Ile Pro Asn Ser Val Leu Ser Thr Glu Ala Gly Val Val Arg
145                 150                 155                 160
Val Thr Ala Pro Gly Thr Lys Tyr Cys Glu Gly Ser Thr Asp Ser Ala
                165                 170                 175
Gly Asp Gly Leu Arg Leu Asp Ala Val Asn Pro Asn Tyr Thr Leu Val
            180                 185                 190
Asn Leu Arg Pro Glu Gly Phe Thr Pro Lys Val Ser Gly Leu Ala Phe
        195                 200                 205
Leu Gly Asn Gly Asp Leu Ala Val Leu Thr Thr Gly Ser Val Asn Ser
    210                 215                 220
Gly Gly Trp Asp Thr Ser Thr Pro Gly Lys Val Phe Val Leu Lys Gly
225                 230                 235                 240
Ala Gln Ala Ala Asp Gly Pro Glu Asp Val Thr Val Val Glu Ala Ala
                245                 250                 255
Gly Gly Leu Leu Asn Pro Met Gly Ile Asp Val Ile Asp Asp Lys Ile
            260                 265                 270
Tyr Val Ser Glu Arg Tyr Gln Leu Thr Glu Leu Ser Asp Thr Asn Gly
        275                 280                 285
Asp Gly Ser Tyr Glu Thr Lys Arg Lys Val Ala Glu Tyr Pro Ser Gly
    290                 295                 300
Asn Asn Phe His Glu Phe Ala Phe Gly Leu Ile His Asp Glu Lys Asn
305                 310                 315                 320

Phe Tyr Val Asn Leu Ser Val Ala Ile Asp Asn Gly Gly Ala Thr Thr
                325                 330                 335
Asn Pro Gln Pro Ala Lys Asn Arg Gly Thr Ser Val Lys Ile Asp Arg
            340                 345                 350
Ala Thr Gly Ala Ile Ser Tyr Val Ala Gly Gly Leu Arg Thr Pro Asn
        355                 360                 365
Gly Val Ala Phe Gly Pro Glu Asn Glu Leu Phe Ala Met Asp Asn Gln
    370                 375                 380
Gly Ala Trp Leu Pro Ala Asn Lys Leu Val Asn Val Lys Gln Asp Arg
385                 390                 395                 400
Phe Phe Asn His Tyr Thr Asn Pro Ala Gly Pro Phe Asp Gln Asn Pro
                405                 410                 415
Val Thr Ala Pro Val Val Trp Ile Pro Gln Asn Glu Ile Gly Asn Ser
            420                 425                 430
Pro Ser Thr Pro Ile Met Leu Lys Asp Gly Pro Phe Ala Gly Gln Met
        435                 440                 445
Met Phe Gly Asp Val Thr Tyr Gly Gly Leu Gln Arg Ala Phe Leu Glu
    450                 455                 460
Lys Val Asp Gly Glu Phe Gln Gly Ala Val Phe Arg His Ser Ala Gly
465                 470                 475                 480
Phe Glu Val Gly Val Asn Arg Val Ile Glu Gly Pro Asp Thr Ser Leu
                485                 490                 495
Tyr Ile Gly Gly Thr Gly Glu Gly Gly Asn Trp Gly Glu Ala Gly Lys
            500                 505                 510
Leu Thr Tyr Gly Leu Gln Lys Leu Val Pro Asn Thr Thr Gln Asn Ala
        515                 520                 525
Phe Asp Met Lys Ser Met Lys Val Val Glu Gly Gly Phe Glu Ile Glu
    530                 535                 540

Tyr Thr Gln Pro Leu Ser Asp Glu Thr Leu Ala Asn Leu Ala Ser Ala
545                 550                 555                 560
Tyr Arg Ala Glu Gln Trp Arg Tyr Leu Pro Thr Ser Thr Tyr Gly Gly
                565                 570                 575
Pro Lys Val Asp Glu Glu Ile Leu Ser Leu Thr Gly Ala Thr Ala Ser
            580                 585                 590
Ala Asp Arg Lys Thr Val Thr Val Ser Phe Asp Gly Leu Lys Gln Gly
        595                 600                 605
Arg Val Val His Leu Arg Ser Pro Gln Pro Phe Ala Ala Ala Ser Gly
    610                 615                 620
Asp Thr Leu Trp Ser Thr Glu Ala Trp Tyr Thr Leu Asn Ser Leu Pro
625                 630                 635                 640
Gly Tyr Val Ser Pro Ala Asp Gln Gly Trp Tyr Glu Ala Glu Glu Ala
                645                 650                 655
Arg Leu Ile Gly Gly Ala Lys Phe Asp Ala Glu His Ser Gly Tyr Ser
            660                 665                 670
Gly Ala Gly Phe Ala Gly Gly Met Trp Gln Ala Gly Ser Ala Phe Glu
        675                 680                 685
Phe Thr Val Asn Ala Glu Thr Ala Gly Thr Val Pro Val Ser Val Arg
    690                 695                 700
Tyr Ser Asn Gly Pro Asn Pro Ala Pro Gly Ser Lys Asp Val Asn Leu
705                 710                 715                 720
Tyr Val Asn Gly Gln Asn Leu Gly Lys Trp Asp Phe Ala Ser Thr Gly
                725                 730                 735
Asp Trp Lys Thr Trp Ala Thr Ile Thr Arg Asp Met Pro Leu Val Ala
            740                 745                 750
Gly Thr Asn Thr Ile Ala Leu Lys Tyr Glu Thr Gly Asn Lys Gly Asn
        755                 760                 765

Ile Asn Val Asp Val Leu Ser Ile Gly Thr Ala Asp Ile Cys Ala Pro
    770                 775                 780
Ser Gln Val Glu Asp Gly Tyr Arg Pro Leu Phe Asp Gly Thr Leu Glu
785                 790                 795                 800
Ser Leu Asn Ala Gly Trp Arg Met Ala Gly Pro Gly Gly Phe Gly Arg
                805                 810                 815
Gln Asn Asp Cys Ser Ile Arg Gly Glu Gly Gly Met Gly Leu Leu Trp
            820                 825                 830
His Lys Ala Gln Glu Leu Asn Glu Tyr Ser Leu Lys Leu Asp Trp Lys
        835                 840                 845
Leu Ile Ala Asp His Asn Gly Gly Val Phe Val Gly Phe Pro Asp Pro
    850                 855                 860
Lys Asn Asp Pro Trp Ile Ala Val Asn Gln Gly Tyr Glu Ile Gln Ile
865                 870                 875                 880
Asp Ala Ser Asp Ala Ala Asp Arg Thr Thr Gly Ala Ile Tyr Thr Phe
                885                 890                 895
Gln Gly Ala Asp Ala Asp Ala Val Lys Ala Ser Leu Lys Pro Val Gly
            900                 905                 910
Gln Trp Asn Ala Tyr Glu Ile Val Val Lys Gly Gln Thr Ile Lys Ile
        915                 920                 925
Phe Leu Asn Gly Thr Leu Val Asn Asp Phe Thr Ser Thr Asp Pro Ala
    930                 935                 940
Arg Asp Leu Ser Gln Gly Phe Ile Gly Leu Gln Asn His Gly Gly Gly
945                 950                 955                 960
Glu Ala Val Ser Tyr Arg Asn Val Arg Val Lys Asp Ile Asp Glu Pro
                965                 970                 975
Ala Pro Leu Ala Val Thr Ala Ser Ala Glu Val Lys Cys Leu Ala Lys
            980                 985                 990

Lys Ala Ser Val Thr Ala Arg Ala Thr Asn Thr Asp Thr Val Pro Val
        995                 1000                1005
Asp Val Thr Leu Thr Thr Ala Trp Gly Glu Arg Val Ile Pro Ala Val
    1010                1015                1020
Gln Pro Gly Ala Thr Val Phe His Thr Phe Thr Thr Arg Ala Ala Ser
1025                1030                1035                1040
Val Pro Ala Gly Glu Ala Thr Val Thr Ala Thr Gly Asp Gly Arg Thr
                1045                1050                1055
Gly Gly Ala Thr Ala Ser Tyr Ala Ala Lys Ser Cys Gly
            1060                1065

<210> 9
<211> 3348
<212> DNA
<213> Microbacterium sp. KNK011

<223> DNA sequence of aldehyde oxidase containing signal peptide
<400> 9
atg ccc gta ccc gct gtc ttg tcc ggc ccc cgc cgg cgc agc acc cgt 48
gtc gcg agg acc ctc gcg gcc ggt ctc ctg ggc gga gca ctc gtg gcc 96
aca gga gcg atc ctg ccc gcc ggt gcc gcc gtc gcc ccc gcc gtc aac 144
acc ccg gcg gac ctg ccc aag caa gaa ccg ggt gtg acg ctt cgc acc 192
tac tcc acc ccg ccc ctc acc gag ctc tgc acg ctg aag tcg ggc cag 240
acc ccg aac gtc gac aag ctc atg tcg acg atc gac tgg cgc acc gac 288
gag cag ttc ggc gcg ggc gac aac ttc atc acg cac gcc ctg gcc aac 336
ctc acc gtc agc acg ccc ggt cag tac gcc ttc cgc ctg acg agc gac 384
gac ggc tca cgc ctc acc ctc gac ggc acg cag ctg atc gac aac gac 432
gga ctg cac gga gcg gaa tcc gtc gag ggc acc gtc acg ctc gac gtg 480

gga gtc cac gac ctg ttc gtc gag atg ttc gag gcc acc aac ggc cag 528
cag ctc acc gtc gaa tgg aag gtc ccg ggc tcc tcg agc ttc acc gtc 576
atc ccc aac agc gtg ctc agc acc gag gcg ggt gtc gtg cgc gtc acc 624
gcc ccc ggg acg aag tac tgc gag ggc agc acc gac tcc gcc ggc gac 672
ggt ctg cgc ctg gat gcc gtg aac ccc aac tac acg ctc gtg aac ctg 720
cgc ccc gag ggc ttc acc ccc aag gtg tcg ggc ctg gcc ttc ctc ggc 768
aac ggc gat ctc gcc gtg ctg acc acc ggc tcg gtc aac tcc ggg gga 816
tgg gac acc tcg acc ccc ggc aag gtc ttc gtg ctg aag ggc gcc cag 864
gcg gcc gac gga ccc gag gac gtc acc gtc gtt gag gcg gcc ggc ggt 912
ctg ctg aac ccg atg ggc atc gac gtc atc gac gac aag atc tac gtc 960
tcg gag cgc tac cag ctc acc gag ctc agc gac acg aac ggc gac ggc 1008
tcg tac gag acg aag cgc aag gtc gcc gag tac ccc tcg ggc aac aac 1056
ttc cac gag ttc gcc ttc ggc ctg atc cac gac gag aag aac ttc tac 1104
gtc aac ctc tcg gtc gcg atc gac aac ggc ggg gcg acc acc aac ccg 1152
cag ccg gcg aag aac cgt ggc acc tcg gtc aag atc gac cgc gcc acc 1200
ggg gcg atc agc tac gtc gcc ggc ggt ctg cgg acc ccc aac ggc gtg 1248
gcc ttc ggt ccc gag aac gaa ctc ttc gcg atg gac aac cag ggt gcc 1296
tgg ctg ccc gcg aac aag ctc gtc aac gtc aag cag gat cgc ttc ttc 1344
aac cac tac acg aac ccc gcg ggc ccg ttc gat cag aac ccc gtc acg 1392
gct ccc gtc gtg tgg atc ccg cag aac gag atc ggc aac tcc ccg agc 1440
acg ccc atc atg ctg aag gac ggt ccc ttc gcg gga cag atg atg ttc 1488
ggc gac gtc acc tac ggc ggc ctg cag cgc gcc ttc ctc gag aag gtg 1536
gac ggt gag ttc cag ggc gcg gtc ttc cgc cac tcc gcg ggc ttc gag 1584
gtc ggc gtc aac cgc gtc atc gag ggc ccc gac acg tcg ctg tac atc 1632
ggc ggc acc gga gaa ggc ggc aac tgg ggt gag gcg ggc aag ctc aca 1680
tac ggc ctg cag aag ctc gtc ccg aac acc acg cag aac gcc ttc gac 1728
atg aaa tcc atg aag gtc gtc gag ggc gga ttc gag atc gag tac acc 1776
cag ccc ctc tcc gac gag acg ctg gcg aac ctc gcc tcg gcg tac cgg 1824

gcc gag cag tgg cgc tac ctg ccc acc tcc acc tac ggc gga ccg aag 1872
gtc gac gag gag atc ctc tcg ctc acc ggc gcg acg gca tcc gcc gac 1920
cgc aag acg gtc acg gtc tca ttc gac ggt ctg aag caa ggc cgc gtc 1968
gtg cac ctg cgc tcg ccg cag ccc ttc gcc gca gcc agt ggc gac acg 2016
ctg tgg agc acc gag gcc tgg tac acg ctc aac tcg ctg ccg ggc tac 2064
gtc tcg ccg gcc gac cag ggc tgg tac gag gcc gag gag gcc cgc ctg 2112
atc ggc ggc gcc aag ttc gac gcc gag cac agc ggt tac tcg gga gcc 2160
ggc ttc gcc ggc ggc atg tgg cag gcc gga tcc gcg ttc gag ttc acg 2208
gtg aac gcc gag acc gcg ggc acc gtc ccc gtc tcg gtg cgc tat tcc 2256
aac ggc ccg aac ccc gcg ccc ggc tcc aag gac gtc aac ctc tac gtc 2304
aac ggt cag aac ctc ggg aag tgg gac ttc gcc tcc acg ggc gac tgg 2352
aag acc tgg gcc acg atc acg cgg gac atg ccg ctg gtc gcc ggg acg 2400
aac acc atc gcg ctg aag tac gag acg ggc aac aag ggc aac atc aac 2448
gtc gac gtc ctg tcg atc ggc acc gcc gac atc tgc gcc ccg tcg cag 2496
gtc gag gac ggc tac cgc ccg ctc ttc gac ggc acg ctc gag agc ctg 2544
aac gcc ggg tgg cgc atg gcc ggc ccc ggc ggc ttc ggt cga cag aac 2592
gac tgc agc atc cgc ggc gaa ggc ggc atg ggc ctg ctc tgg cac aag 2640
gcg cag gag ctg aac gag tac agc ctc aag ctg gac tgg aag ctc atc 2688
gcc gat cac aac ggc ggc gtc ttc gtc ggg ttc ccc gac ccg aag aac 2736
gac ccg tgg atc gcg gtc aac cag ggc tac gag atc cag atc gac gcg 2784
tcc gac gcc gcc gat cgc acc acc ggt gcg atc tac acc ttc cag ggt 2832
gcg gat gcc gac gcg gtg aag gcc tcg ctc aag ccc gtc ggt cag tgg 2880
aac gcg tac gag atc gtc gtg aag ggg cag acc atc aag atc ttc ctc 2928
aac ggc acg ctg gtg aac gac ttc acc agc acc gat ccc gct cgc gac 2976
ctc tcg cag ggc ttc atc ggc ctg cag aac cac ggc ggt ggg gag gcg 3024
gtg tcg tac cgc aac gtc cgc gtc aag gac atc gac gag ccg gcc ccc 3072
ctc gcg gtg acc gcc tcg gcc gag gtc aag tgc ctg gcc aag aag gcg 3120
tcg gtg acg gcc cgc gcg acc aac acc gac acg gtg ccg gtc gac gtg 3168

acg ctc acg acg gcg tgg ggc gag cgg gtc atc ccc gcc gtg cag ccc 3216
ggg gcg acg gtg ttc cac acc ttc acc acc cgc gcc gcg tcc gtc ccc 3264
gcg ggc gag gcg acg gtg acc gcg acc ggc gac ggt cgc acc ggc ggg 3312
gcc acg gcc tcg tac gca gcc aag agc tgc ggc tga 3348

<210> 10
<211> 3210
<212> DNA
<213> Microbacterium sp. KNK011

<223> DNA sequence of aldehyde oxidase

<400> 10
gtc aac acc ccg gcg gac ctg ccc aag caa gaa ccg ggt gtg acg ctt 48
cgc acc tac tcc acc ccg ccc ctc acc gag ctc tgc acg ctg aag tcg 96
ggc cag acc ccg aac gtc gac aag ctc atg tcg acg atc gac tgg cgc 144
acc gac gag cag ttc ggc gcg ggc gac aac ttc atc acg cac gcc ctg 192
gcc aac ctc acc gtc agc acg ccc ggt cag tac gcc ttc cgc ctg acg 240
agc gac gac ggc tca cgc ctc acc ctc gac ggc acg cag ctg atc gac 288
aac gac gga ctg cac gga gcg gaa tcc gtc gag ggc acc gtc acg ctc 336
gac gtg gga gtc cac gac ctg ttc gtc gag atg ttc gag gcc acc aac 384
ggc cag cag ctc acc gtc gaa tgg aag gtc ccg ggc tcc tcg agc ttc 432
acc gtc atc ccc aac agc gtg ctc agc acc gag gcg ggt gtc gtg cgc 480
gtc acc gcc ccc ggg acg aag tac tgc gag ggc agc acc gac tcc gcc 528
ggc gac ggt ctg cgc ctg gat gcc gtg aac ccc aac tac acg ctc gtg 576
aac ctg cgc ccc gag ggc ttc acc ccc aag gtg tcg ggc ctg gcc ttc 624
ctc ggc aac ggc gat ctc gcc gtg ctg acc acc ggc tcg gtc aac tcc 672

ggg gga tgg gac acc tcg acc ccc ggc aag gtc ttc gtg ctg aag ggc 720
gcc cag gcg gcc gac gga ccc gag gac gtc acc gtc gtt gag gcg gcc 768
ggc ggt ctg ctg aac ccg atg ggc atc gac gtc atc gac gac aag atc 816
tac gtc tcg gag cgc tac cag ctc acc gag ctc agc gac acg aac ggc 864
gac ggc tcg tac gag acg aag cgc aag gtc gcc gag tac ccc tcg ggc 912
aac aac ttc cac gag ttc gcc ttc ggc ctg atc cac gac gag aag aac 960
ttc tac gtc aac ctc tcg gtc gcg atc gac aac ggc ggg gcg acc acc 1008
aac ccg cag ccg gcg aag aac cgt ggc acc tcg gtc aag atc gac cgc 1056
gcc acc ggg gcg atc agc tac gtc gcc ggc ggt ctg cgg acc ccc aac 1104
ggc gtg gcc ttc ggt ccc gag aac gaa ctc ttc gcg atg gac aac cag 1152
ggt gcc tgg ctg ccc gcg aac aag ctc gtc aac gtc aag cag gat cgc 1200
ttc ttc aac cac tac acg aac ccc gcg ggc ccg ttc gat cag aac ccc 1248
gtc acg gct ccc gtc gtg tgg atc ccg cag aac gag atc ggc aac tcc 1296
ccg agc acg ccc atc atg ctg aag gac ggt ccc ttc gcg gga cag atg 1344
atg ttc ggc gac gtc acc tac ggc ggc ctg cag cgc gcc ttc ctc gag 1392
aag gtg gac ggt gag ttc cag ggc gcg gtc ttc cgc cac tcc gcg ggc 1440
ttc gag gtc ggc gtc aac cgc gtc atc gag ggc ccc gac acg tcg ctg 1488
tac atc ggc ggc acc gga gaa ggc ggc aac tgg ggt gag gcg ggc aag 1536
ctc aca tac ggc ctg cag aag ctc gtc ccg aac acc acg cag aac gcc 1584
ttc gac atg aaa tcc atg aag gtc gtc gag ggc gga ttc gag atc gag 1632
tac acc cag ccc ctc tcc gac gag acg ctg gcg aac ctc gcc tcg gcg 1680
tac cgg gcc gag cag tgg cgc tac ctg ccc acc tcc acc tac ggc gga 1728
ccg aag gtc gac gag gag atc ctc tcg ctc acc ggc gcg acg gca tcc 1776
gcc gac cgc aag acg gtc acg gtc tca ttc gac ggt ctg aag caa ggc 1824
cgc gtc gtg cac ctg cgc tcg ccg cag ccc ttc gcc gca gcc agt ggc 1872
gac acg ctg tgg agc acc gag gcc tgg tac acg ctc aac tcg ctg ccg 1920
ggc tac gtc tcg ccg gcc gac cag ggc tgg tac gag gcc gag gag gcc 1968
cgc ctg atc ggc ggc gcc aag ttc gac gcc gag cac agc ggt tac tcg 2016

gga gcc ggc ttc gcc ggc ggc atg tgg cag gcc gga tcc gcg ttc gag 2064
ttc acg gtg aac gcc gag acc gcg ggc acc gtc ccc gtc tcg gtg cgc 2112
tat tcc aac ggc ccg aac ccc gcg ccc ggc tcc aag gac gtc aac ctc 2160
tac gtc aac ggt cag aac ctc ggg aag tgg gac ttc gcc tcc acg ggc 2208
gac tgg aag acc tgg gcc acg atc acg cgg gac atg ccg ctg gtc gcc 2256
ggg acg aac acc atc gcg ctg aag tac gag acg ggc aac aag ggc aac 2304
atc aac gtc gac gtc ctg tcg atc ggc acc gcc gac atc tgc gcc ccg 2352
tcg cag gtc gag gac ggc tac cgc ccg ctc ttc gac ggc acg ctc gag 2400
agc ctg aac gcc ggg tgg cgc atg gcc ggc ccc ggc ggc ttc ggt cga 2448
cag aac gac tgc agc atc cgc ggc gaa ggc ggc atg ggc ctg ctc tgg 2496
cac aag gcg cag gag ctg aac gag tac agc ctc aag ctg gac tgg aag 2544
ctc atc gcc gat cac aac ggc ggc gtc ttc gtc ggg ttc ccc gac ccg 2592
aag aac gac ccg tgg atc gcg gtc aac cag ggc tac gag atc cag atc 2640
gac gcg tcc gac gcc gcc gat cgc acc acc ggt gcg atc tac acc ttc 2688
cag ggt gcg gat gcc gac gcg gtg aag gcc tcg ctc aag ccc gtc ggt 2736
cag tgg aac gcg tac gag atc gtc gtg aag ggg cag acc atc aag atc 2784
ttc ctc aac ggc acg ctg gtg aac gac ttc acc agc acc gat ccc gct 2832
cgc gac ctc tcg cag ggc ttc atc ggc ctg cag aac cac ggc ggt ggg 2880
gag gcg gtg tcg tac cgc aac gtc cgc gtc aag gac atc gac gag ccg 2928
gcc ccc ctc gcg gtg acc gcc tcg gcc gag gtc aag tgc ctg gcc aag 2976
aag gcg tcg gtg acg gcc cgc gcg acc aac acc gac acg gtg ccg gtc 3024
gac gtg acg ctc acg acg gcg tgg ggc gag cgg gtc atc ccc gcc gtg 3072
cag ccc ggg gcg acg gtg ttc cac acc ttc acc acc cgc gcc gcg tcc 3120
gtc ccc gcg ggc gag gcg acg gtg acc gcg acc ggc gac ggt cgc acc 3168
ggc ggg gcc acg gcc tcg tac gca gcc aag agc tgc ggc tga 3210

<210> 11
<211> 1107
<212> PRT
<213> Cellulosimicrobium cellulans IFO 15516

<223> amino acid sequence of aldehyde oxidase containing signal peptide

<400> 11
Met Leu Glu Arg Thr Ser Leu Pro Val Ser Arg Arg Ile Arg Tyr Arg
1               5                   10                  15
Arg Gly Ala Ala Ala Leu Thr Leu Gly Ala Val Val Val Ala Gly Trp
            20                  25                  30
Ala Val Pro Ala Ala Ala Asp Leu Pro Glu Gln Glu Pro Gly Val Thr
        35                  40                  45
Leu Arg Thr Phe Gln Leu Ala Gln Asn Pro Gly Ala Val Cys Thr Leu
    50                  55                  60
Lys Ser Gly Gln Thr Pro Asn Val Asp Lys Leu Met Pro Thr Ile Asp
65                  70                  75                  80
Trp Ser Thr Ala Glu Gln Phe Gly Ala Glu Asp Asn Phe Ile Ser Gln
                85                  90                  95
Val Ser Ala Asn Leu His Val Pro Ala Asp Gly Gln Tyr Gln Phe Arg
            100                 105                 110
Val Thr Asn Asp Asp Gly Ala Leu Val Tyr Ile Asp Gly Gln Leu Val
        115                 120                 125
Val Glu Asn Asp Gly Pro Asn Asp Ser Thr Ser Val Glu Gly Ser Ala
    130                 135                 140
Thr Leu Thr Ala Gly Val His Asp Leu Arg Val Asp Tyr Tyr Glu Gly
145                 150                 155                 160

Ser Asp Lys Gln Arg Leu Thr Leu Ala Trp Lys Thr Pro Gly Ser Ser
                165                 170                 175
Thr Phe Glu Val Ile Pro Thr Ser Ala Leu Ser Thr Glu Ala Gly Val
            180                 185                 190
Val Arg Val Thr Ala Pro Gly Tyr Lys Tyr Cys Glu Gly Ala Thr Asp
        195                 200                 205
Thr Ala Gly Asp Gly Leu Arg Leu Asp Ser Val Asn Pro Asn Tyr Asp
    210                 215                 220
Leu Val Asp Leu Arg Pro Glu Gly Phe Glu Pro Lys Val Ser Gly Leu
225                 230                 235                 240
Ala Phe Thr Pro Asp Glu Lys Leu Ala Val Val Thr Thr Gly Glu Val
                245                 250                 255
Ser Ser Gly Gly Trp Arg Pro Asp Pro Val Ser Gly Glu Val Tyr Phe
            260                 265                 270
Leu Asp Gly Val Leu Asp Ala Asp Gly Pro Glu Asp Val Thr Ala Thr
        275                 280                 285
Lys Val Ala Asp Glu Leu Leu Asn Pro Met Gly Ile Glu Val Val Glu
    290                 295                 300
Asp Ser Ile Phe Val Ser Glu Arg Tyr Gln Leu Thr Gln Leu Thr Asp
305                 310                 315                 320
Pro Asp Gly Asp Gly Phe Tyr Asp Thr His Thr Lys Ile Ala Glu Trp
                325                 330                 335
Pro Asp Gly Gly Asn Phe His Glu Phe Ala Phe Gly Leu Ile His Asp
            340                 345                 350
Glu Asp Tyr Phe Tyr Val Asn Leu Ser Val Ala Ile Asn Asn Gly Gly
        355                 360                 365
Ala Thr Thr Asn Pro Gln Pro Ala Ala Asn Arg Gly Thr Ser Ile Lys
    370                 375                 380

Ile Asp Arg Glu Thr Gly Glu Val Thr Tyr Val Ala Gly Gly Leu Arg
385                 390                 395                 400
Thr Pro Asn Gly Ile Gly Phe Gly Pro Glu Asp Glu Ile Phe Ala Thr
                405                 410                 415
Asp Asn Gln Gly Ala Trp Leu Pro Ser Asn Lys Leu Ile His Ile Gln
            420                 425                 430
Gln Asp Lys Phe Tyr Asn His Tyr Thr Asn Pro Ala Gly Pro Phe Asp
        435                 440                 445
Ala Asn Pro Val Thr Pro Pro Ala Val Trp Leu Pro Gln Asn Glu Ile
    450                 455                 460
Ala Asn Ser Pro Gly Asn Pro Ile Leu Val Glu Asp Gly Glu Phe Ala
465                 470                 475                 480
Gly Gln Met Leu Leu Gly Asp Val Thr Tyr Gly Gly Ile Gln Arg Ala
                485                 490                 495
Phe Leu Glu Lys Val Asp Gly Glu Phe Gln Gly Ala Val Phe Arg His
            500                 505                 510
Thr Ala Gly Leu Glu Val Gly Val Asn Arg Val Ile Tyr Gly Pro Asp
        515                 520                 525
Gly Ala Leu Tyr Ala Gly Gly Thr Gly Glu Gly Gly Asn Trp Gly Glu
    530                 535                 540
Ser Gly Lys Leu Arg Tyr Gly Leu Gln Lys Leu Val Pro Val Asn Glu
545                 550                 555                 560
Asp Ser Phe Asp Met Lys Glu Met Arg Val Val Glu Gly Gly Phe Glu
                565                 570                 575
Ile Glu Tyr Thr Asp Pro Val Ser Asp Glu Val Val Glu Lys Leu Ala
            580                 585                 590
Asp Ala Tyr Gln Val Lys Gln Trp Arg Tyr Val Pro Thr Gln Gln Tyr
        595                 600                 605

Gly Gly Pro Lys Val Asp Glu Glu Pro Leu Phe Val Thr Asp Ala Thr
    610                 615                 620
Val Ser Glu Asp Arg Thr Thr Val Thr Leu Thr Ile Asp Gly Leu Lys
625                 630                 635                 640
Pro Asp His Val Val Tyr Ile Arg Ser Pro Arg Pro Phe Ala Ser Ala
                645                 650                 655
Glu Gly Thr Glu Leu Leu Ser Thr Glu Ala Trp Tyr Thr Leu Asn Ser
            660                 665                 670
Leu Pro Gly Tyr Val Ala Pro Ala Asp Arg Gly Trp Tyr Glu Ala Glu
        675                 680                 685
Leu Ala Gln Pro Leu Gly Ser Ser Ser Ile Gly Ser Asp His Ser Asn
    690                 695                 700
Tyr Ser Gly Ser Gly Phe Ala Ala Gly Met Thr Asn Val Gly Ala Gly
705                 710                 715                 720
Arg Thr Phe Ser Val Thr Val Pro Glu Ala Gly Thr Tyr Pro Val Asn
                725                 730                 735
Val Arg Tyr Ala Asn Gly Ile His Pro Tyr Thr Ser Leu Arg Ser Lys
            740                 745                 750
Asn Val Ser Leu His Val Asn Gly Gln Asp Leu Gly Gln Trp Asn Phe
        755                 760                 765
Pro Thr Thr Gly Ser Trp Lys Asp Trp Gly Val Gln Thr Arg Asp Leu
    770                 775                 780
Gln Leu Gln Ala Gly Thr Asn Thr Ile Thr Leu Ala Tyr Glu Ala Gly
785                 790                 795                 800
Asp Glu Gly Asn Ile Asn Ile Asp Val Leu Ser Ile Gly Glu Asn Pro
                805                 810                 815
Asp Ile Cys Ser Pro Gly Glu Val Glu Glu Gly Tyr Thr Ala Ile Tyr
            820                 825                 830

Asp Gly Thr Leu Ala Ser Leu Gln Glu Gly Trp Arg Met Ala Gly Pro
        835                 840                 845
Gly Gly Phe Gly Arg Gln Glu Asp Cys Ser Ile Arg Gly Ala Gly Gly
    850                 855                 860
Met Gly Leu Leu Trp Tyr Asp Gln Glu Leu Gly Glu Asn Tyr Ser Leu
865                 870                 875                 880
Lys Leu Asp Trp Lys Leu Thr Lys Asp Asp Asn Gly Gly Val Phe Val
                885                 890                 895
Gly Phe Pro Asn Pro Gly Asp Asp Pro Trp Val Ala Val Asn Lys Gly
            900                 905                 910
Tyr Glu Ile Gln Ile Asp Ala Thr Asp Ala Asp Asp Arg Thr Thr Gly
        915                 920                 925
Ala Val Tyr Thr Phe Gln Gly Ala Asp Glu Ala Ala Arg Asp Ala Ala
    930                 935                 940
Leu Lys Pro Val Gly Gln Trp Asn Ala Tyr Asp Ile Arg Val Glu Gly
945                 950                 955                 960
Asp Arg Ile Arg Ile Tyr Leu Asn Asp Val Leu Val Asn Asp Phe Thr
                965                 970                 975
Ser Thr Asp Pro Ala Arg Leu Val Asn Ser Phe Val Gly Ile Gln Asn
            980                 985                 990
His Gly Ser Gly Glu Met Val Asn Tyr Arg Asn Ile Arg Phe Lys Glu
        995                 1000                1005
Leu Thr Asp Glu Pro Val Glu Glu Leu Ala Ile Ser Thr Thr Val Gln
    1010                1015                1020
Thr Arg Cys Leu Ala Gly Lys Val Tyr Val Ala Val Arg Ala Thr Asn
1025                1030                1035                1040
Asp Asp Thr Val Pro Ala Asp Ile Thr Leu Thr Thr Pro Phe Gly Thr
                1045                1050                1055

Lys Thr Val Thr Gly Val Gln Pro Gly Ala Ser Ala Tyr Gln Ser Phe
            1060                1065                1070
Ala Ser Arg Ser Thr Ser Val Glu Ala Gly Thr Ala Gln Val Ser Ala
        1075                1080                1085
Thr Gly Gly Asp Leu Thr Phe Gln Ala Asp Val Ala Tyr Glu Ala Ala
    1090                1095                1100
Ser Cys Gly
1105

<210> 12
<211> 1069
<212> PRT
<213> Cellulosimicrobium cellulans IFO 15516

<223> amino acid sequence of aldehyde oxidase

<400> 12
Asp Leu Pro Glu Gln Glu Pro Gly Val Thr Leu Arg Thr Phe Gln Leu
  1               5                  10                  15
Ala Gln Asn Pro Gly Ala Val Cys Thr Leu Lys Ser Gly Gln Thr Pro
                20                  25                  30
Asn Val Asp Lys Leu Met Pro Thr Ile Asp Trp Ser Thr Ala Glu Gln
            35                  40                  45
Phe Gly Ala Glu Asp Asn Phe Ile Ser Gln Val Ser Ala Asn Leu His
        50                  55                  60
Val Pro Ala Asp Gly Gln Tyr Gln Phe Arg Val Thr Asn Asp Asp Gly
 65                  70                  75                  80

Ala Leu Val Tyr Ile Asp Gly Gln Leu Val Val Glu Asn Asp Gly Pro
                85                  90                  95
Asn Asp Ser Thr Ser Val Glu Gly Ser Ala Thr Leu Thr Ala Gly Val
            100                 105                 110
His Asp Leu Arg Val Asp Tyr Tyr Glu Gly Ser Asp Lys Gln Arg Leu
        115                 120                 125
Thr Leu Ala Trp Lys Thr Pro Gly Ser Ser Thr Phe Glu Val Ile Pro
    130                 135                 140
Thr Ser Ala Leu Ser Thr Glu Ala Gly Val Val Arg Val Thr Ala Pro
145                 150                 155                 160
Gly Tyr Lys Tyr Cys Glu Gly Ala Thr Asp Thr Ala Gly Asp Gly Leu
                165                 170                 175
Arg Leu Asp Ser Val Asn Pro Asn Tyr Asp Leu Val Asp Leu Arg Pro
            180                 185                 190
Glu Gly Phe Glu Pro Lys Val Ser Gly Leu Ala Phe Thr Pro Asp Glu
        195                 200                 205
Lys Leu Ala Val Val Thr Thr Gly Glu Val Ser Ser Gly Gly Trp Arg
    210                 215                 220
Pro Asp Pro Val Ser Gly Glu Val Tyr Phe Leu Asp Gly Val Leu Asp
225                 230                 235                 240
Ala Asp Gly Pro Glu Asp Val Thr Ala Thr Lys Val Ala Asp Glu Leu
                245                 250                 255
Leu Asn Pro Met Gly Ile Glu Val Val Glu Asp Ser Ile Phe Val Ser
            260                 265                 270
Glu Arg Tyr Gln Leu Thr Gln Leu Thr Asp Pro Asp Gly Asp Gly Phe
        275                 280                 285
Tyr Asp Thr His Thr Lys Ile Ala Glu Trp Pro Asp Gly Gly Asn Phe
    290                 295                 300

His Glu Phe Ala Phe Gly Leu Ile His Asp Glu Asp Tyr Phe Tyr Val
305                 310                 315                 320
Asn Leu Ser Val Ala Ile Asn Asn Gly Gly Ala Thr Thr Asn Pro Gln
                325                 330                 335
Pro Ala Ala Asn Arg Gly Thr Ser Ile Lys Ile Asp Arg Glu Thr Gly
            340                 345                 350
Glu Val Thr Tyr Val Ala Gly Gly Leu Arg Thr Pro Asn Gly Ile Gly
        355                 360                 365
Phe Gly Pro Glu Asp Glu Ile Phe Ala Thr Asp Asn Gln Gly Ala Trp
    370                 375                 380
Leu Pro Ser Asn Lys Leu Ile His Ile Gln Gln Asp Lys Phe Tyr Asn
385                 390                 395                 400
His Tyr Thr Asn Pro Ala Gly Pro Phe Asp Ala Asn Pro Val Thr Pro
                405                 410                 415
Pro Ala Val Trp Leu Pro Gln Asn Glu Ile Ala Asn Ser Pro Gly Asn
            420                 425                 430
Pro Ile Leu Val Glu Asp Gly Glu Phe Ala Gly Gln Met Leu Leu Gly
        435                 440                 445
Asp Val Thr Tyr Gly Gly Ile Gln Arg Ala Phe Leu Glu Lys Val Asp
    450                 455                 460
Gly Glu Phe Gln Gly Ala Val Phe Arg His Thr Ala Gly Leu Glu Val
465                 470                 475                 480
Gly Val Asn Arg Val Ile Tyr Gly Pro Asp Gly Ala Leu Tyr Ala Gly
                485                 490                 495
Gly Thr Gly Glu Gly Gly Asn Trp Gly Glu Ser Gly Lys Leu Arg Tyr
            500                 505                 510
Gly Leu Gln Lys Leu Val Pro Val Asn Glu Asp Ser Phe Asp Met Lys
        515                 520                 525

Glu Met Arg Val Val Glu Gly Gly Phe Glu Ile Glu Tyr Thr Asp Pro
    530                 535                 540
Val Ser Asp Glu Val Val Glu Lys Leu Ala Asp Ala Tyr Gln Val Lys
545                 550                 555                 560
Gln Trp Arg Tyr Val Pro Thr Gln Gln Tyr Gly Gly Pro Lys Val Asp
                565                 570                 575
Glu Glu Pro Leu Phe Val Thr Asp Ala Thr Val Ser Glu Asp Arg Thr
            580                 585                 590
Thr Val Thr Leu Thr Ile Asp Gly Leu Lys Pro Asp His Val Val Tyr
        595                 600                 605
Ile Arg Ser Pro Arg Pro Phe Ala Ser Ala Glu Gly Thr Glu Leu Leu
    610                 615                 620
Ser Thr Glu Ala Trp Tyr Thr Leu Asn Ser Leu Pro Gly Tyr Val Ala
625                 630                 635                 640
Pro Ala Asp Arg Gly Trp Tyr Glu Ala Glu Leu Ala Gln Pro Leu Gly
                645                 650                 655
Ser Ser Ser Ile Gly Ser Asp His Ser Asn Tyr Ser Gly Ser Gly Phe
            660                 665                 670
Ala Ala Gly Met Thr Asn Val Gly Ala Gly Arg Thr Phe Ser Val Thr
        675                 680                 685
Val Pro Glu Ala Gly Thr Tyr Pro Val Asn Val Arg Tyr Ala Asn Gly
    690                 695                 700
Ile His Pro Tyr Thr Ser Leu Arg Ser Lys Asn Val Ser Leu His Val
705                 710                 715                 720
Asn Gly Gln Asp Leu Gly Gln Trp Asn Phe Pro Thr Thr Gly Ser Trp
                725                 730                 735
Lys Asp Trp Gly Val Gln Thr Arg Asp Leu Gln Leu Gln Ala Gly Thr
            740                 745                 750

Asn Thr Ile Thr Leu Ala Tyr Glu Ala Gly Asp Glu Gly Asn Ile Asn
        755                 760                 765
Ile Asp Val Leu Ser Ile Gly Glu Asn Pro Asp Ile Cys Ser Pro Gly
    770                 775                 780
Glu Val Glu Glu Gly Tyr Thr Ala Ile Tyr Asp Gly Thr Leu Ala Ser
785                 790                 795                 800
Leu Gln Glu Gly Trp Arg Met Ala Gly Pro Gly Gly Phe Gly Arg Gln
                805                 810                 815
Glu Asp Cys Ser Ile Arg Gly Ala Gly Gly Met Gly Leu Leu Trp Tyr
            820                 825                 830
Asp Gln Glu Leu Gly Glu Asn Tyr Ser Leu Lys Leu Asp Trp Lys Leu
        835                 840                 845
Thr Lys Asp Asp Asn Gly Gly Val Phe Val Gly Phe Pro Asn Pro Gly
    850                 855                 860
Asp Asp Pro Trp Val Ala Val Asn Lys Gly Tyr Glu Ile Gln Ile Asp
865                 870                 875                 880
Ala Thr Asp Ala Asp Asp Arg Thr Thr Gly Ala Val Tyr Thr Phe Gln
                885                 890                 895
Gly Ala Asp Glu Ala Ala Arg Asp Ala Ala Leu Lys Pro Val Gly Gln
            900                 905                 910
Trp Asn Ala Tyr Asp Ile Arg Val Glu Gly Asp Arg Ile Arg Ile Tyr
        915                 920                 925
Leu Asn Asp Val Leu Val Asn Asp Phe Thr Ser Thr Asp Pro Ala Arg
    930                 935                 940
Leu Val Asn Ser Phe Val Gly Ile Gln Asn His Gly Ser Gly Glu Met
945                 950                 955                 960
Val Asn Tyr Arg Asn Ile Arg Phe Lys Glu Leu Thr Asp Glu Pro Val
                965                 970                 975

Glu Glu Leu Ala Ile Ser Thr Thr Val Gln Thr Arg Cys Leu Ala Gly
            980                 985                 990
Lys Val Tyr Val Ala Val Arg Ala Thr Asn Asp Asp Thr Val Pro Ala
        995                 1000                1005
Asp Ile Thr Leu Thr Thr Pro Phe Gly Thr Lys Thr Val Thr Gly Val
    1010                1015                1020
Gln Pro Gly Ala Ser Ala Tyr Gln Ser Phe Ala Ser Arg Ser Thr Ser
1025                1030                1035                1040
Val Glu Ala Gly Thr Ala Gln Val Ser Ala Thr Gly Gly Asp Leu Thr
                1045                1050                1055
Phe Gln Ala Asp Val Ala Tyr Glu Ala Ala Ser Cys Gly
            1060                1065

<210> 13
<211> 3324
<212> DNA
<213> Cellulosimicrobium cellulans IFO 15516

<223> DNA sequence of aldehyde oxidase containing signal peptide

<400> 13
atg ctc gaa cgc acc agc ctg ccg gtg tcc cgc cgg atc agg tac cga 48
cgc ggc gcg gcc gcc ctg acc ctt ggc gcc gtg gtc gtc gcg ggc tgg 96
gcc gtc ccc gcc gcg gcc gac ctc ccc gag cag gag ccc ggc gtc acg 144
ctg cgg acc ttc cag ctc gcg cag aac ccc ggg gcg gtc tgc acc ctg 192
aag tcc ggc cag acc ccc aac gtc gac aag ctc atg ccg acc atc gac 240
tgg tcg acg gcc gag cag ttc ggc gcg gag gac aac ttc atc tcg cag 288

gtg agc gcg aac ctg cac gtc ccc gcg gac ggc cag tac cag ttc cgc 336
gtg acc aac gac gac ggc gcg ctc gtc tac atc gac ggc cag ctc gtg 384
gtc gag aac gac ggc ccg aac gac tcg acc tcc gtc gag ggc agc gcg 432
acg ctc acc gcg ggc gtg cac gac ctg cgg gtc gac tac tac gag ggc 480
agc gac aag cag cgc ctc acg ctc gcg tgg aag acg ccg ggc agc tcg 528
acg ttc gag gtg atc ccg acg tcg gcg ctc agc acc gag gcg ggc gtc 576
gtg cgc gtg acc gcg ccc ggc tac aag tac tgc gag ggc gcg acc gac 624
acg gcc ggc gac ggc ctg cgt ctc gac agc gtc aac ccc aac tac gac 672
ctc gtc gac ctg cgc ccc gag ggc ttc gag ccc aag gtc tcg ggc ctg 720
gcc ttc acg ccg gac gag aag ctc gcc gtc gtg acg acc ggc gag gtc 768
agc tcc ggc ggg tgg cgc ccc gac ccg gtg tcc ggc gag gtg tac ttc 816
ctc gac ggc gtc ctg gac gcg gac ggc ccc gag gac gtc acg gcg acg 864
aag gtc gcg gac gag ctg ctc aac ccg atg ggc atc gag gtc gtc gag 912
gac tcg atc ttc gtc tcg gag cgg tac cag ctc acg cag ctc acc gac 960
cct gac ggc gac ggc ttc tac gac acg cac acg aag atc gcg gag tgg 1008
ccc gac ggc ggc aac ttc cac gag ttc gcg ttc ggc ctg atc cac gac 1056
gag gac tac ttc tac gtc aac ctc tcc gtg gcc atc aac aac ggc ggc 1104
gcg acg acc aac ccg cag ccg gcg gcc aac cgc ggc acg tcg atc aag 1152
atc gac cgc gag acg ggt gag gtc acg tac gtc gcg ggc ggt ctc cgc 1200
acg ccg aac ggc atc ggc ttc ggc ccc gag gac gag atc ttc gcg acg 1248
gac aac cag ggt gcc tgg ctc ccg tcg aac aag ctc atc cac atc cag 1296
cag gac aag ttc tac aac cac tac acg aac ccg gcc ggg ccg ttc gac 1344
gcg aac ccc gtc acc ccg ccg gcc gtg tgg ctc ccg cag aac gag atc 1392
gcc aac tcc ccg ggc aac ccg atc ctc gtc gag gac ggc gag ttc gcc 1440
ggc cag atg ctg ctc ggc gac gtg acc tac ggc ggc atc cag cgc gcg 1488
ttc ctc gag aag gtc gac ggc gag ttc cag ggt gcg gtc ttc cgc cac 1536
acc gcg ggc ctc gag gtc ggc gtg aac cgt gtc atc tac ggt ccc gac 1584
ggc gcg ctc tac gcc ggc ggc acc ggt gag ggc ggc aac tgg ggc gag 1632

tcc ggc aag ctc cgg tac ggc ctc cag aag ctc gtc ccc gtg aac gag 1680
gac tcc ttc gac atg aag gag atg cgc gtg gtc gag ggc ggc ttc gag 1728
atc gag tac acc gac ccg gtg tcc gac gag gtc gtc gag aag ctg gcc 1776
gac gcg tac cag gtc aag cag tgg cgc tac gtg ccg acg cag cag tac 1824
ggc ggc ccg aag gtc gac gag gag ccc ctc ttc gtc acc gac gcc acc 1872
gtg tcc gag gac cgc acg acc gtc acg ctg acg atc gac ggc ctc aag 1920
ccc gac cac gtc gtc tac atc cgc tcg ccg cgc ccg ttc gcg tcc gcc 1968
gag ggc acc gag ctc ctc agc acc gag gcc tgg tac acg ctc aac tcg 2016
ctg ccc ggc tac gtc gcc ccg gcc gac cgc ggc tgg tac gag gcg gag 2064
ctg gcc cag ccg ctc ggc agc tcg agc atc ggc tcg gac cac agc aac 2112
tac tcc ggt tcg ggc ttc gcc gcg ggc atg acg aac gtc ggt gcc ggc 2160
cgc acg ttc tcc gtg acc gtc ccc gag gcg ggc acc tac ccg gtc aac 2208
gtg cgc tac gcc aac ggc atc cac ccg tac acg tcg ctg cgg tcg aag 2256
aac gtc tcg ctc cac gtg aac ggc cag gac ctg ggc cag tgg aac ttc 2304
ccc acc acg gga agc tgg aag gac tgg ggc gtg cag acg cgc gac ctc 2352
cag ctc cag gcc ggc acg aac acc atc acg ctc gcg tac gag gcc ggt 2400
gac gag ggc aac atc aac atc gac gtc ctg tcg atc ggc gag aac ccg 2448
gac atc tgc tcc ccg ggc gag gtc gag gag ggc tac acc gcg atc tac 2496
gac ggc acc ctc gcg agc ctc cag gag ggc tgg cgc atg gcc ggt ccg 2544
ggt ggc ttc ggc cgc cag gag gac tgc tcg atc cgc ggt gcg ggt ggc 2592
atg ggc ctg ctc tgg tac gac cag gag ctc ggc gag aac tac agc ctg 2640
aag ctc gac tgg aag ctc acg aag gac gac aac ggc ggc gtg ttc gtc 2688
ggg ttc ccg aac ccg ggc gac gac ccg tgg gtg gcc gtc aac aag ggc 2736
tac gag atc cag atc gac gcg acg gac gcc gac gac cgc acc acc ggc 2784
gcg gtc tac acg ttc cag ggc gcc gac gag gca gcg cgc gac gcc gcc 2832
ctc aag ccg gtc ggg cag tgg aac gcg tac gac atc cgt gtc gag gga 2880
gac cgc atc cgc atc tac ctc aac gac gtg ctc gtc aac gac ttc acg 2928
agc acc gac ccg gcc cgg ctg gtg aac agc ttc gtc ggc atc cag aac 2976

cac ggc agc ggc gag atg gtc aac tac cgg aac atc cgg ttc aag gag 3024
ctg acc gac gag ccg gtc gag gag ctc gcg atc tcg acg acg gtc cag 3072
acc cgc tgc ctg gcg ggc aag gtc tac gtc gcg gtc cgt gcc acg aac 3120
gac gac acg gtg ccg gcg gac atc acg ctg acg acg ccg ttc ggg acg 3168
aag acg gtg acc ggc gtc cag ccg ggt gcc tcc gcg tac cag tcg ttc 3216
gcg tcg cgc tcg acc tcg gtc gag gcg ggc acc gcc cag gtg tcc gcg 3264
acg ggc ggc gac ctg acg ttc cag gcg gac gtc gcc tac gag gcc gcg 3312
tcc tgc ggc tga 3324

<210> 14
<211> 3210
<212> DNA
<213> Cellulosimicrobium cellulans IFO 15516

<223> DNA sequence of aldehyde oxidase

<400> 14
gac ctc ccc gag cag gag ccc ggc gtc acg ctg cgg acc ttc cag ctc 48
gcg cag aac ccc ggg gcg gtc tgc acc ctg aag tcc ggc cag acc ccc 96
aac gtc gac aag ctc atg ccg acc atc gac tgg tcg acg gcc gag cag 144
ttc ggc gcg gag gac aac ttc atc tcg cag gtg agc gcg aac ctg cac 192
gtc ccc gcg gac ggc cag tac cag ttc cgc gtg acc aac gac gac ggc 240
gcg ctc gtc tac atc gac ggc cag ctc gtg gtc gag aac gac ggc ccg 288
aac gac tcg acc tcc gtc gag ggc agc gcg acg ctc acc gcg ggc gtg 336
cac gac ctg cgg gtc gac tac tac gag ggc agc gac aag cag cgc ctc 384
acg ctc gcg tgg aag acg ccg ggc agc tcg acg ttc gag gtg atc ccg 432
acg tcg gcg ctc agc acc gag gcg ggc gtc gtg cgc gtg acc gcg ccc 480

ggc tac aag tac tgc gag ggc gcg acc gac acg gcc ggc gac ggc ctg 528
cgt ctc gac agc gtc aac ccc aac tac gac ctc gtc gac ctg cgc ccc 576
gag ggc ttc gag ccc aag gtc tcg ggc ctg gcc ttc acg ccg gac gag 624
aag ctc gcc gtc gtg acg acc ggc gag gtc agc tcc ggc ggg tgg cgc 672
ccc gac ccg gtg tcc ggc gag gtg tac ttc ctc gac ggc gtc ctg gac 720
gcg gac ggc ccc gag gac gtc acg gcg acg aag gtc gcg gac gag ctg 768
ctc aac ccg atg ggc atc gag gtc gtc gag gac tcg atc ttc gtc tcg 816
gag cgg tac cag ctc acg cag ctc acc gac cct gac ggc gac ggc ttc 864
tac gac acg cac acg aag atc gcg gag tgg ccc gac ggc ggc aac ttc 912
cac gag ttc gcg ttc ggc ctg atc cac gac gag gac tac ttc tac gtc 960
aac ctc tcc gtg gcc atc aac aac ggc ggc gcg acg acc aac ccg cag 1008
ccg gcg gcc aac cgc ggc acg tcg atc aag atc gac cgc gag acg ggt 1056
gag gtc acg tac gtc gcg ggc ggt ctc cgc acg ccg aac ggc atc ggc 1104
ttc ggc ccc gag gac gag atc ttc gcg acg gac aac cag ggt gcc tgg 1152
ctc ccg tcg aac aag ctc atc cac atc cag cag gac aag ttc tac aac 1200
cac tac acg aac ccg gcc ggg ccg ttc gac gcg aac ccc gtc acc ccg 1248
ccg gcc gtg tgg ctc ccg cag aac gag atc gcc aac tcc ccg ggc aac 1296
ccg atc ctc gtc gag gac ggc gag ttc gcc ggc cag atg ctg ctc ggc 1344
gac gtg acc tac ggc ggc atc cag cgc gcg ttc ctc gag aag gtc gac 1392
ggc gag ttc cag ggt gcg gtc ttc cgc cac acc gcg ggc ctc gag gtc 1440
ggc gtg aac cgt gtc atc tac ggt ccc gac ggc gcg ctc tac gcc ggc 1488
ggc acc ggt gag ggc ggc aac tgg ggc gag tcc ggc aag ctc cgg tac 1536
ggc ctc cag aag ctc gtc ccc gtg aac gag gac tcc ttc gac atg aag 1584
gag atg cgc gtg gtc gag ggc ggc ttc gag atc gag tac acc gac ccg 1632
gtg tcc gac gag gtc gtc gag aag ctg gcc gac gcg tac cag gtc aag 1680
cag tgg cgc tac gtg ccg acg cag cag tac ggc ggc ccg aag gtc gac 1728
gag gag ccc ctc ttc gtc acc gac gcc acc gtg tcc gag gac cgc acg 1776
acc gtc acg ctg acg atc gac ggc ctc aag ccc gac cac gtc gtc tac 1824

atc cgc tcg ccg cgc ccg ttc gcg tcc gcc gag ggc acc gag ctc ctc 1872
agc acc gag gcc tgg tac acg ctc aac tcg ctg ccc ggc tac gtc gcc 1920
ccg gcc gac cgc ggc tgg tac gag gcg gag ctg gcc cag ccg ctc ggc 1968
agc tcg agc atc ggc tcg gac cac agc aac tac tcc ggt tcg ggc ttc 2016
gcc gcg ggc atg acg aac gtc ggt gcc ggc cgc acg ttc tcc gtg acc 2064
gtc ccc gag gcg ggc acc tac ccg gtc aac gtg cgc tac gcc aac ggc 2112
atc cac ccg tac acg tcg ctg cgg tcg aag aac gtc tcg ctc cac gtg 2160
aac ggc cag gac ctg ggc cag tgg aac ttc ccc acc acg gga agc tgg 2208
aag gac tgg ggc gtg cag acg cgc gac ctc cag ctc cag gcc ggc acg 2256
aac acc atc acg ctc gcg tac gag gcc ggt gac gag ggc aac atc aac 2304
atc gac gtc ctg tcg atc ggc gag aac ccg gac atc tgc tcc ccg ggc 2352
gag gtc gag gag ggc tac acc gcg atc tac gac ggc acc ctc gcg agc 2400
ctc cag gag ggc tgg cgc atg gcc ggt ccg ggt ggc ttc ggc cgc cag 2448
gag gac tgc tcg atc cgc ggt gcg ggt ggc atg ggc ctg ctc tgg tac 2496
gac cag gag ctc ggc gag aac tac agc ctg aag ctc gac tgg aag ctc 2544
acg aag gac gac aac ggc ggc gtg ttc gtc ggg ttc ccg aac ccg ggc 2592
gac gac ccg tgg gtg gcc gtc aac aag ggc tac gag atc cag atc gac 2640
gcg acg gac gcc gac gac cgc acc acc ggc gcg gtc tac acg ttc cag 2688
ggc gcc gac gag gca gcg cgc gac gcc gcc ctc aag ccg gtc ggg cag 2736
tgg aac gcg tac gac atc cgt gtc gag gga gac cgc atc cgc atc tac 2784
ctc aac gac gtg ctc gtc aac gac ttc acg agc acc gac ccg gcc cgg 2832
ctg gtg aac agc ttc gtc ggc atc cag aac cac ggc agc ggc gag atg 2880
gtc aac tac cgg aac atc cgg ttc aag gag ctg acc gac gag ccg gtc 2928
gag gag ctc gcg atc tcg acg acg gtc cag acc cgc tgc ctg gcg ggc 2976
aag gtc tac gtc gcg gtc cgt gcc acg aac gac gac acg gtg ccg gcg 3024
gac atc acg ctg acg acg ccg ttc ggg acg aag acg gtg acc ggc gtc 3072
cag ccg ggt gcc tcc gcg tac cag tcg ttc gcg tcg cgc tcg acc tcg 3120
gtc gag gcg ggc acc gcc cag gtg tcc gcg acg ggc ggc gac ctg acg 3168

ttc cag gcg gac gtc gcc tac gag gcc gcg tcc tgc ggc tga 3210

<210> 15
<211> 27
<212> PRT
<213> Streptomyces sp. KNK269

<223> N-terminal amino acid sequence of subunit gamma of aldehyde oxidas
e

<400> 15
Gly Ala Asp Leu Glu Pro Glu Pro Val Val Asp Glu His Ser Thr Val
1               5                   10                  15
Thr Leu Asn Val Asn Gly Asp Pro Thr Thr Leu
            20                  25

<210> 16
<211> 40
<212> PRT
<213> Streptomyces sp. KNK269

<223> N-terminal amino acid sequence of subunit beta of aldehyde oxidase

<400> 16
Met Lys Pro Phe Gly Tyr Val Arg Pro Ala Ser Pro Asp Glu Ala Val
1               5                   10                  15

Arg Leu Cys Ala Glu Gly Ser Gly Ala Arg Phe Leu Gly Gly Gly Thr
            20                  25                  30
Asn Leu Val Asp Leu Met Lys Leu
        35                  40

<210> 17
<211> 36
<212> PRT
<213> Streptomyces sp. KNK269

<223> amino acid sequence (from Ala231 to Ala266) of subunit beta of ald
ehyde oxidase

<400> 17
Ala Arg Asp Arg Ala Ser Phe Ala Phe Ala Leu Val Ser Val Ala Ala
1               5                   10                  15
Thr Leu Arg Val Asp Ala Gly Arg Val Glu His Ala Thr Leu Ala Phe
            20                  25                  30
Gly Gly Val Ala
        35

<210> 18
<211> 24
<212> PRT
<213> Streptomyces sp. KNK269

<223> N-terminal amino acid sequence of subunit alpha of aldehyde oxidas
e

<400> 18
Ala Pro Gln Phe Pro Gly Ala Pro Val Val Arg Arg Glu Ala Arg Asp
1               5                   10                  15
Lys Val Thr Gly Thr Ala Arg Tyr
            20

<210> 19
<211> 38
<212> PRT
<213> Streptomyces sp. KNK269

<223> amino acid sequence (from Leu261to Glu298) of subunit alpha of ald
ehyde oxidase

<400> 19
Leu Val Phe Pro Arg Ala Gln Leu Ala Glu Val Val Gly His Arg Ala
1               5                   10                  15
Pro Thr Ile Gln Arg Val Arg Leu Gly Ala Asp Leu Asp Gly Val Leu
            20                  25                  30
Thr Ala Val Ser His Glu
        35

<210> 20

<211> 35

<212> PRT

<213> Streptomyces sp. KNK269

<223> amino acid sequence (from Gly659 to Thr693) of subunit alpha of al
dehyde oxidase

<400> 20
Gly Ile Gly Glu Ile Gly Ile Val Gly Thr Ala Ala Ala Ile Gly Asn
1               5                   10                  15
Ala Val Arg His Ala Thr Gly Ala Arg Leu Arg Glu Leu Pro Leu Thr
            20                  25                  30
Thr Asp Thr
        35

<210> 21
<211> 29
<212> DNA
<213> Artificial Sequence

<220>
<223> mixed DNA primer (1) corresponding to N-terminal amino acid sequen
ce of subunit gamma of aldehyde oxidase of Streptomyces sp. KNK269

<400> 21
gacctsgarc csgarccsgt sgtsgacga   29

<210> 22
<211> 23
<212> DNA
<213> Artificial Sequence

<220>
<223> complementary mixed DNA primer (2) corresponding to amino acid se
quence (from Asp251 to Ala258) of subunit beta of Streptomyces sp. KNK26
9 aldehyde oxidase

<400> 22
gcgtgytcsa cscgbccsgc gtc 23

<210> 23
<211> 23
<212> DNA
<213> Artificial Sequence

<220>
<223> mixed DNA primer (3) corresponding to amino acid sequence (from A
sp251 to Ala258) of subunit beta of Streptomyces sp. KNK269 aldehyde oxi
dase

<400> 23
gacgcsggvc gsgtsgarca cgc 23

<210> 24
<211> 23
<212> DNA
<213> Artificial Sequence

<220>
<223> complementary mixed DNA primer (4) corresponding to amino acid seq
uence (from Leu274 to Glu281) of subunit alpha of Streptomyces sp. KNK26
9 aldehyde oxidase
<400> 24
cgctggatsg tsggsgcscg gtg  23

<210> 25
<211> 23
<212> DNA
<213> Artificial Sequence

<220>
<223> mixed DNA primer (5) corresponding to amino acid sequence (from Le
u274 to Glu281) of subunit alpha of Streptomyces sp. KNK269 aldehyde oxi
dase

<400> 25
caccgsgcsc csacsatcca gcg  23

<210> 26
<211> 23
<212> DNA
<213> Artificial Sequence

<220>
<223>  complementary mixed DNA primer (6) corresponding to amino acid se
quence (from Leu686 to Glu693) of subunit alpha of Streptomyces sp. KNK2
69 aldehyde oxidase
<400> 26
gtgtcsgtsg tsagsggsag ytc  23



<210> 27
<211> 10
<212> PRT
<213> Microbacterium sp. KNK011

<223> N-terminal amino acid sequence of aldehyde oxidase

<400> 27
 Val Asn Thr Pro Ala Asp Leu Pro Lys Gln
   1               5                   10



<210> 28
<211> 34
<212> PRT

<213> Microbacterium sp. KNK011

<223> internal amino acid sequence of aldehyde oxidase

<400> 28
Val Asp Gly Glu Phe Gln Gly Ala Val Phe Arg His Ser Ala Gly Phe
1               5                   10                  15
Thr Val Gly Val Asn Arg Val Ile Glu Gly Pro Asp Thr Ser Leu Tyr
            20                  25                  30
Ile Gly

<210> 29
<211> 19
<212> PRT
<213> Microbacterium sp. KNK011

<223> internal amino acid sequence of aldehyde oxidase

<400> 29
Ile Phe Leu Asn Gly Thr Leu Val Asn Asp Phe Thr Ser Val Asp Pro
1               5                   10                  15
Ala Arg Glu

<210> 30
<211> 29
<212> DNA

<213> Artificial Sequence

<220>
<223>  mixed DNA primer (1) corresponding to N-terminal amino acid seque
nce of aldehydeoxidase from Microbacterium sp. KNK011
<400> 30
gtcaacacsc csgcngayct bccsaarca  29

<210> 31
<211> 26
<212> DNA
<213> Artificial Sequence

<220>
<223>  complementary mixed DNA primer (2)corresponding to amino acid seq
uence (from Asp513 to Phe521) of aldehyde oxidase from Microbacterium sp
. KNK011
<400> 31
aasacbgcsc cctgraaytc nccrtc  26

<210> 32
<211> 26
<212> DNA
<213> Artificial Sequence

<220>

<223> mixed DNA primer (3) corresponding to amino acid sequence (from A
sp513 to Phe521) of aldehydeoxidase from Microbacterium sp. KNK011
<400> 32
gacggsgart tccagggvgc ngtntt 26

<210> 33
<211> 32
<212> DNA
<213> Artificial Sequence

<220>
<223> complementary mixed DNA primer (4) corresponding to amino acid se
quence (from Phe959 to Thr969) of aldehyde oxidase from Microbacterium s
p. KNK011
<400> 33
gtgaagtcgt tsacsagsgt gccrttnagr aa 32

<210> 34
<211> 32
<212> DNA
<213> Artificial Sequence

<220>
<223> DNA primer containing Nde I restriction site for cloning of aldeh
yde oxidase from Microbacterium sp. KNK011

<400> 34
gtgccgccgt cgcccatatg gtcaacaccc cg 32

<210> 35
<211> 29
<212> DNA
<213> Artificial Sequence

<220>
<223> DNA primer containing EcoR I restriction site for cloning of alde
hyde oxidase from Microbacterium sp. KNK011

<400> 35
ggagagcatg gggaattctg ggacatgag 29

<210> 36
<211> 23
<212> PRT
<213> Cellulosimicrobium cellulans IFO 15516

<223> N-terminal amino acid sequence of aldehyde oxidase

<400> 36
Asp Leu Pro Glu Gln Glu Pro Gly Val Thr Leu Arg Thr Phe Gln Leu
1               5                   10                  15

Ala Gln Asn Pro Thr Ala Val
            20

<210> 37
<211> 31
<212> PRT
<213> Cellulosimicrobium cellulans IFO 15516

<223> internal amino acid sequence of aldehyde oxidase

<400> 37
Lys Thr Pro Gly Ser Thr Val Phe Glu Val Ile Pro Thr Ser Ala Leu
1               5                   10                  15
Ser Thr Glu Ala Gly Val Val Tyr Val Thr Ala Pro Gly Val Lys
            20                  25                  30

<210> 38
<211> 34
<212> PRT
<213> Cellulosimicrobium cellulans IFO 15516

<223> internal amino acid sequence of aldehyde oxidase

<400> 38
Lys Ile Ala Glu Trp Pro Asp Gly Gly Asn Phe His Glu Ile Ala Phe
1               5                   10                  15

Glu Leu Ile His Asp Glu Asp Tyr Phe Tyr Val Asn Leu Ser Val Ala
            20                  25                  30
Ile Asn

<210> 39
<211> 25
<212> DNA
<213> Artificial Sequence

<220>
<223>  mixed DNA primer (1) corresponding to N-terminal amino acid seque
nce of aldehydeoxidase from Cellulosimicrobium cellulans IFO 15516

<400> 39
acctsccsga gcaggagccs ggsgt  25

<210> 40
<211> 26
<212> DNA
<213> Artificial Sequence

<220>
<223>  complementary mixed DNA primer (2) corresponding to amino acid se
quence (from Ile350 to Val358) of aldehyde oxidase from Cellulosimicrobi
um cellulans IFO 15516

<400> 40
acgtagaagt agtcctcgtc gtggat 26

<210> 41
<211> 23
<212> DNA
<213> Artificial Sequence

<220>
<223> mixed DNA primer (3) corresponding to amino acid sequence (from T
hr176 to Thr183) of aldehydeoxidase from Cellulosimicrobium cellulans IF
0 15516

<400> 41
acsgtsttcg aggtsatccc sac 23

<210> 42
<211> 25
<212> DNA
<213> Artificial Sequence

<220>
<223> complementary DNA primer (4) corresponding to DNA sequence (2521-
2545) of aldehyde oxidase from MIicrobacterium sp. KNK011
<400> 42
tcaggctctc gagcgtgccg tcgaa 25

## Description

### Technical Field

The present invention relates to a process for producing glyoxylic acid from glyoxal using microorganisms and/or enzymes derived from microorganisms. Glyoxylic acid is used as a synthetic raw material for vanillin, ethyl vanillin, or the like. This compound is useful as an intermediate for synthesizing agricultural chemicals and pharmaceuticals.

### Background Art

As a process for producing glyoxylic acid, chemical methods such as nitric acid oxidation of glyoxal has conventionally been known. At present, almost all of glyoxylic acids are produced by such chemical methods. However, chemical methods such as nitric acid oxidation of glyoxal are likely to generate by-products such as organic acids other than glyoxylic acid. Such by-products affect the quality of the produced glyoxylic acid. In order to remove these by-products, complicated steps are required. In addition, the treatment of a large amount of salt waste, which is produced during the neutralization step of a large amount of nitric acid used, has been problematic.

Examples of a process for biochemical production of glyoxylic acid may include: a process for converting glycolic acid into glyoxylic acid using glycolate oxidase derived from plants (see National Publication of International Patent Application Nos. 7-502895 and 8-508159); and a process for converting glycolic acid into glyoxylic acid using microorganisms (see Japanese Patent Laid-Open Nos. 7-163380 and 8-322581). However, a process for biochemical synthesis of glyoxylic acid from glyoxal, which is an inexpensively available compound that is easily synthesized from ethylene glycol or acetaldehyde and which is used as a material in the chemical synthetic method of glyoxylic acid, has not yet been reported.

Moreover, it has been confirmed that oxidase that oxidizes an aldehyde group exists in animals, plants, or the like. However, it has not been reported that such an enzyme exhibits activity to glyoxal. Furthermore, it has been known that several types of white-rot fungi such as *Phanerochaete chrysosporium* produce an enzyme having activity of reacting with glyoxal to generate hydrogen peroxide (which is called glyoxal oxidase) to outside of the microbial cells (hereinafter referred to briefly as "cells"), thereof (see Journal of Bacteriology, (1987), 169, 2195-2201; and Pro. Natl. Acad. Sci. (1990), 87, 2936-2940). However, a product produced from glyoxal during the oxidization reaction of the glyoxal with the enzyme that is produced by the above white-rot fungi has not yet been identified. Since the enzyme derived from these wood-rotting fungi has oxidization activity to glyoxylic acid, which is the same level as that to glyoxal, it is difficult that glyoxal is converted into glyoxylic acid and it is then accumulated, using the above enzyme. Other than the enzyme derived from the wood-rotting fungi, no oxidase derived from microorganisms that oxidizes glyoxal has been reported.

Thus, it is an object of the present invention to provide a microorganism having activity of converting glyoxal into glyoxylic acid and/or an enzyme having the above activity, and a process for efficient production of glyoxylic acid using these items.

### Disclosure of the Invention

As a result of intensive studies directed towards developing a process for efficient production of glyoxylic acid, the present inventors have found a microorganism having activity of converting glyoxal into glyoxylic acid. The present inventors have studied in detail the synthesis of glyoxylic acid using such a microorganism and/or a solution that contains enzyme obtained from the above microorganism, thereby completing the present invention.

That is to say, the present invention relates to a process for production of glyoxylic acid, which is characterized in that the process comprises allowing oxidoreductase that can convert glyoxal into glyoxylic acid, or at least one or a mixture consisting of two or more types selected from the group consisting of a culture broth, a supernatant of the culture broth, cells, and a processed product of a microorganism that can produce the above described oxidoreductase, to act on glyoxal, so as to convert glyoxal into glyoxylic acid.

The above-described oxidoreductase is preferably oxidase.

The above-described oxidoreductase is preferably an enzyme obtained from at least one microorganism selected from the group consisting of the genus Stenotrophomonas, Streptomyces, Pseudomonas, Microbacterium, Achromobacter, Cellulomonas, Cellulosimicrobium, and Morganella.

The above described microorganism is preferably *Stenotrophomonas sp.* KNK235 (deposit institution: National Institute of Advanced Industrial Science and Technology; address: AIST Tsukuba, Central 6, Higashi 1-1-1, Tsukuba, Ibaraki, Japan (postal code: 305-8566); deposit date: September 6, 2002; accession No. FERM P-19002), *Streptomyces sp.* KNK269 (deposit institution: National Institute of Advanced Industrial Science and Technology; address: AIST Tsukuba, Central 6, Higashi 1-1-1, Tsukuba, Ibaraki, Japan (postal code: 305-8566); deposit date: September 6, 2002; accession No. FERM BP-08556), *Pseudomonas sp.* KNK058 (deposit institution: National Institute of Advanced Industrial Science and Technology; address: AIST Tsukuba, Central 6, Higashi 1-1-1, Tsukuba, Ibaraki, Japan (postal code: 305-8566); deposit date: December 13, 2002; accession No. FERM BP-08555), *Pseudomonas sp*. KNK254 (deposit institution: National Institute of Advanced Industrial Science and Technology; address: AIST Tsukuba, Central 6, Higashi 1-1-1, Tsukuba, Ibaraki, Japan (postal code: 305-8566); deposit date: September 6, 2002; accession No. FERM P-19003), *Microbacterium sp.* KNK011 (deposit institution: National Institute of Advanced Industrial Science and Technology; address: AIST Tsukuba, Central 6, Higashi 1-1-1, Tsukuba, Ibaraki, Japan (postal code: 305-8566); deposit date: December 13, 2002; accession No. FERM BP-08554), *Achromobacter sp.* IFO 13495 (deposit institution: National Institute of Technology and Evaluation, Biological Resource Center (NBRC); address: Kazusa Kamatari 2-5-8, Kisarazu, Chiba, Japan (postal code: 292-0818)), *Cellulomonas sp*. JCM 2471 (deposit institution: Riken Bioresource Center, Japan Collection of Microorganisms (JCM); address: Hirosawa 2-1, Wako, Saitama, Japan (postal code: 351-0198)), *Cellulomonas turbata* IFO 15012 (deposit institution: National Institute of Technology and Evaluation, Biological Resource Center (NBRC); address: Kazusa Kamatari 2-5-8, Kisarazu, Chiba, Japan (postal code: 292-0818)), *Cellulomonas turbata* IFO 15014 (deposit institution: National Institute of Technology and Evaluation, Biological Resource Center (NBRC); address: Kazusa Kamatari 2-5-8, Kisarazu, Chiba, Japan (postal code: 292-0818)), *Cellulomonas turbata* IFO 15015 (deposit institution: National Institute of Technology and Evaluation, Biological Resource Center (NBRC); address: Kazusa Kamatari 2-5-8, Kisarazu, Chiba, Japan (postal code: 292-0818)), *Cellulosimicrobium cellulans* IFO 15013 (deposit institution: National Institute of Technology and Evaluation, Biological Resource Center (NBRC); address: Kazusa Kamatari 2-5-8, Kisarazu, Chiba, Japan (postal code: 292-0818)), *Cellulosimicrobium cellulans* IFO 15516 (deposit institution: National Institute of Technology and Evaluation, Biological Resource Center (NBRC); address: Kazusa Kamatari 2-5-8, Kisarazu, Chiba, Japan (postal code: 292-0818)), *Cellulosimicrobium cellulans* JCM 6201 (deposit institution: Riken Bioresource Center, Japan Collection of Microorganisms (JCM); address: Hirosawa 2-1, Wako, Saitama, Japan (postal code: 351-0198)), or *Morganella morganii* IFO 3848 (deposit institution: National Institute of Technology and Evaluation, Biological Resource Center (NBRC); address: Kazusa Kamatari 2-5-8, Kisarazu, Chiba, Japan (postal code: 292-0818)).

In the above-described process for production of glyoxylic acid, catalase is preferably allowed to coexist during the reaction.

In addition, the present invention relates to an aldehyde oxidase derived from a microorganism that acts on glyoxal to generate glyoxylic acid.

The activity of the above described aldehyde oxidase to glyoxylic acid is preferably one-tenth or less of the above activity to glyoxal.

The above described aldehyde oxidase is preferably produced by at least one microorganism selected from the group consisting of the genus Stenotrophomonas, Streptomyces Pseudomonas, Microbacterium, Achromobacter, Cellulomonas, Cellulosimicrobium, and Morganella.

The above described microorganism is preferably *Stenotrophomonas sp.* KNK235 (FERM P-19002), *Streptomyces sp.* KNK269 (FERM BP-08556), *Pseudomonas sp.* KNK058 (FERM BP-08555), *Pseudomonas sp.* KNK254 (FERM P-19003), *Microbacterium sp.* KNK011 (FERM BP-08554), *Achromobacter sp.* IFO 13495, *Cellulomonas sp.* JCM 2471, *Cellulomonas turbata* IFO 15012, *Cellulomonas turbata* IFO 15014, *Cellulomonas turbata* IFO 15015, *Cellulosimicrobium cellulans* IFO 15013, *Cellulosimicrobium cellulans* IFO 15516, *Cell ulosimi crobi um cellulans* JCM 6201, or *Morganella morganii* IFO 3848.

The above described aldehyde oxidase is preferably produced by a microorganism belonging to the genus Streptomyces and preferably has the following physicochemical properties (1) to (3):
(1) optimum pH: 6 to 9;
(2) heat stability: the aldehyde oxidase retains activity of 90% or more after it has been treated at pH 7.2 at 60°C for 20 minutes; and
(3) molecular weight: the aldehyde oxidase has a molecular weight of approximately 110,000 in gel filtration analysis, and has three subunit proteins with molecular weights of approximately 25,000, approximately 35,000, and approximately 80,000 in SDS-polyacrylamide gel electrophoresis analysis.

The above described aldehyde oxidase is preferably produced by a microorganism belonging to the genus Pseudomonas and preferably has the following physicochemical properties (1) to (3):
(1) molecular weight: approximately 150, 000 in gel filtration analysis;
(2) optimum reaction temperature: 60°C to 70°C; and
(3) optimum reaction pH: 5 to 7.

The above described aldehyde oxidase is preferably generated by a microorganism belonging to the genus Microbacterium inside and outside of the cells thereof, and preferably has the following physicochemical properties:
molecular weight: a single protein has a molecular weight of approximately 110,000 in SDS-polyacrylamide gel electrophoresis analysis.

The above described aldehyde oxidase is produced by a microorganism belonging to the genus Cellulosimicrobium inside and outside of the cells thereof, and preferably has the following physicochemical properties:
molecular weight: a single protein has a molecular weight of approximately 90,000 to 100,000 in SDS-polyacrylamide gel electrophoresis analysis.

The above-described microorganism belonging to the genus Streptomyces is preferably *Streptomyces sp.* KNK269 (FERM BP-08556).

The above-described microorganism belonging to the genus Pseudomonas is preferably *Pseudomonas sp.* KNK058 (FERM BP-08555).

The above-described microorganism belonging to the genus Microbacterium is preferably *Microbacterium sp.* KNK011 (FERM BP-08554).

The above-described microorganism belonging to the genus Cellulosimicrobium is preferably *Cellulosimicrobium cellulans* IFO 15516.

The above described aldehyde oxidase preferably has a protein described in the following (a) or (b) as a subunit:
(a) a protein having an amino acid sequence represented by SEQ ID NO: 1, 2, or 3; or
(b) a protein comprising an amino acid sequence resulting from deletion, substitution, or addition of one or several amino acids in the amino acid sequence (a).

The above described aldehyde oxidase preferably has a protein encoded by the DNA described in the following (a) or (b) as a subunit:
(a) DNA having a nucleotide sequence represented by SEQ ID NO: 4, 5, or 6; or
(b) DNA which hybridizes with any one DNA consisting of a nucleotide sequence that is complementary to the DNA consisting of the nucleotide sequence (a) under stringent conditions.

The above described aldehyde oxidase preferably has the amino acid sequence described in the following (a) or (b):
(a) an amino acid sequence represented by SEQ ID NO: 7, 8, 11, or 12; or
(b) an amino acid sequence resulting from deletion, substitution, or addition of one or several amino acids in the amino acid sequence (a).

The above described aldehyde oxidase is preferably encoded by the DNA described in the following (a) or (b):
(a) DNA consisting of a nucleotide sequence represented by SEQ ID NO: 9, 10, 13, or 14; or
(b) DNA which hybridizes with DNA consisting of a nucleotide sequence that is complementary to the DNA consisting of the nucleotide sequence (a) under stringent conditions.

Moreover, the present invention also relates to DNA encoding the above described aldehyde oxidase.

Specifically, the above described DNA is preferably DNA encoding a subunit of the above described aldehyde oxidase, which comprises the DNA described in the following (a) or (b) :
(a) DNA having a nucleotide sequence represented by SEQ ID NO: 4, 5, or 6; or
(b) DNA which hybridizes with any one DNA consisting of a nucleotide sequence that is complementary to the DNA consisting of the nucleotide sequence is (a) under stringent conditions.

The above described DNA is preferably DNA encoding the above described aldehyde oxidase, which comprises the DNA described in the following (a) or (b):
(a) DNA having a nucleotide sequence represented by SEQ ID NO: 9, 10, 13, or 14; or
(b) DNA which hybridizes with any one DNA consisting of a nucleotide sequence that is complementary to the DNA consisting of the nucleotide sequence (a) under stringent conditions.

The above described DNA is preferably DNA encoding a subunit of the above described aldehyde oxidase, which comprises an acid sequence resulting from deletion, substitution, or addition of one or several amino acids in the amino acid sequence represented by SEQ ID NO: 1, 2, or 3.

The above described DNA is preferably DNA encoding the above described aldehyde oxidase, which comprises an amino acid sequence resulting from deletion, substitution, or addition of one or several amino acids in the amino acid sequence represented by SEQ ID NO: 7, 8, 11, or 12.

### Brief Description of the Drawings

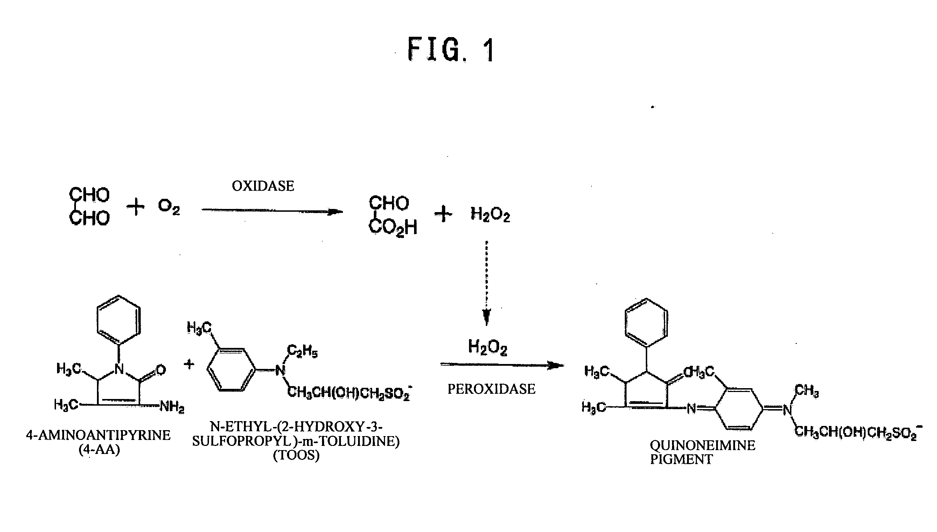

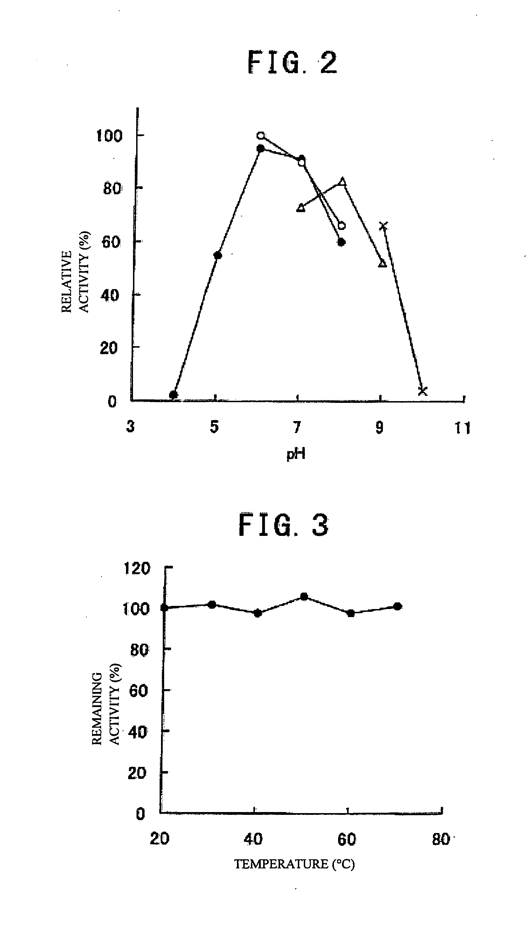

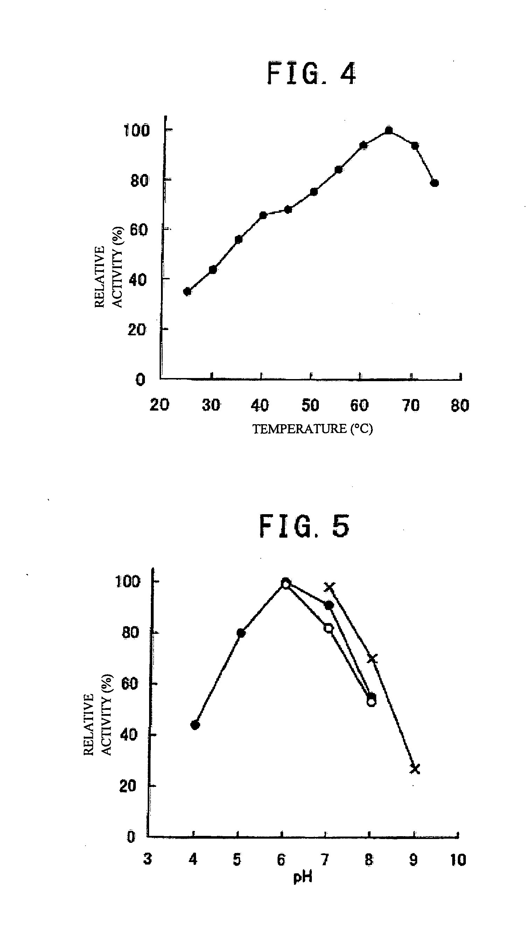

Figure 1 is a view showing the principle of a process for measuring activity in an oxidase reaction. Figure 2 is a view showing the optimum reaction pH of the enzyme derived from the KNK269 strain of the present invention. A 0.1 M MacIlvine buffer (●, filled circle), a 0.1 M phosphate buffer (○, open circle), a 0.1 M Tricine buffer (Δ, triangle), or a 0.1 M Glycine-HCl buffer (X) was used as a buffer. Figure 3 is a view showing the heat stability of the enzyme derived from the KNK269 strain of the present invention. Figure 4 is a view showing the optimum reaction temperature of the enzyme derived from the KNK058 strain of the present invention. Figure 5 is a view showing the optimum reaction pH of the KNK058 strain of the present invention. A 0.1 M MacIlvine buffer (●, filled circle), a 0.1 M phosphate buffer (○, open circle), or a 0.1 M Tricine buffer (x) was used as a buffer.

### Best Mode for Carrying Out the Invention

The conversion reaction of glyoxal into glyoxylic acid of the present invention is shown in the following formulae 1 and 2:

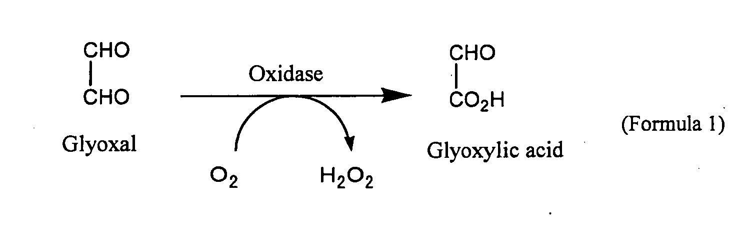

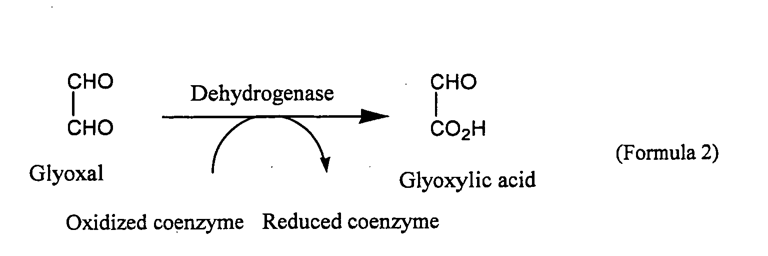

The reaction represented by the above formula 1 takes place when oxidase or a microorganism containing the oxidase intervenes. The reaction represented by the above formula 2 takes place when dehydrogenase or a microorganism containing the dehydrogenase intervenes. In the present specification, conversion of glyoxal into glyoxylic acid includes both the conversion due to the reaction represented by the above formula 1 and the conversion due to the reaction represented by the above formula 2. Accordingly, the term "oxidoreductase that can convert glyoxal into glyoxylic acid" is used to mean oxidase or dehydrogenase. Such oxidoreductase may be either oxidase or dehydrogenase, as long as it is an enzyme converting aldehyde into carboxylic acid. In terms of accumulation of glyoxylic acid, an enzyme that does not have activity or has only low activity to glyoxylic acid is particularly preferable.

Moreover, it is also possible to convert glyoxal into glyoxylic acid using at least one or a mixture consisting of two or more selected from the group consisting of a culture broth, a supernatant of the culture broth, cells, and a processed product of a microorganism that can produce the above oxidoreductase. When the reaction is catalyzed by dehydrogenase (formula 2), a coenzyme (for example, NAD (nicotinamide adenine dinucleotide) or NADP (nicotinamide adenine dinucleotide phosphate)) is necessary, as well as a substrate. On the other hand, when the reaction is catalyzed by oxidase (formula 1), if oxygen exists as well as glyoxal used as a substrate, the reaction progresses. Accordingly, the use of oxidase is advantageous in terms of cost reduction.

When the reaction is catalyzed by oxidase, not only glyoxylic acid but also hydrogen peroxide is produced. Oxidase activity of interest can easily be detected by detecting such hydrogen peroxide. As shown in Figure 1, in the present invention, detection and quantification of the oxidase activity of interest can be carried out by allowing hydrogen peroxide produced as a result of an oxidization reaction to react with 4-aminoantipyrine (hereinafter referred to as 4-AA) and N-ethyl- (2-hydroxy-3-sulfopropyl)-m-toluidine (hereinafter referred to as TOOS), and then detecting and quantifying a quinoneimine pigment produced.

Specifically, 0.1 ml of cell suspension or enzyme solution is added to 0.9 ml of a 100 mM phosphate buffer (pH 7) having the composition indicated below, and an increase in the absorbance at a wavelength of 555 nm is measured at 30°C. In the present invention, enzyme activity for generating 1 µmol H₂O₂ per minute is defined as 1 unit.

| (Composition) | |
|---|---|
| Glyoxal | 20 mM |
| 4-AA | 0.67 mM |
| TOOS | 1.09 mM |
| Peroxidase derived from horseradish (hereinafter referred to as POD) | 2 U/mL |

Quantification of glyoxal and glyoxylic acid can be carried out by high performance liquid chromatography. Analysis by high performance liquid chromatography can be carried out, for example, using a Bio-Rad Aminex HPX-87H column (7.8 mm x 300 mm), also using a 5 mM H₂SO₄ aqueous solution as a solvent, at a flow rate of 0.4 ml/min. Detection is carried out by measuring absorbance at 230 nm or refractive index. Under the present conditions, glyoxal is eluted at 16 minutes, and glyoxylic acid is eluted at 15 minutes.

Microorganisms having oxidase activity of interest can be obtained by the following screening, for example. 0.2 ml of a supernatant obtained by suspending 2 g each of a soil sample collected from several regions in Japan in 10 ml of a saline solution was added to 5 ml of an S medium (pH 7) that had been sterilized by autoclaving (121°C, 20 minutes), which consisted of 10 g of glyoxal, ethylene glycol, propylene glycol, or glycolaldehyde used as a carbon source, 2 g of ammonium nitrate, 1 g of dipotassium hydrogen phosphate, 1g of sodium dihydrogen phosphate, 0.1 g of yeast extract, 0.2 g of magnesium sulfate heptahydrate, and 0.1 g of calcium chloride dihydrate (all of which were amounts contained in 1 liter). It was subjected to enrichment culture at 28°C for 3 to 7 days. 0.1 ml each of a culture broth, in which cells had grown, was spreaded onto S medium plate containing 2% agar. It was then inoculated at 28°C for 3 to 7 days. Thereafter, growing colonies were subjected to static culture using S medium plate containing 2% agar medium again. Strains, the growth of which had been confirmed, were defined as assimilating strains for each carbon source. Thereafter, these assimilating strains were examined in terms of activity of converting glyoxal into glyoxylic acid. Each strain was subjected to culture in 5 ml of S medium placed in a test tube at 28°C with reciprocal shaking for 3 to 5 days. Thereafter, cells were collected by centrifugation, were washed with a saline solution, and were then suspended in 0.5 ml of 100 mM Tris-HCl buffer (pH 8). 0.1 ml of the cell suspension was added to 0.2 ml of 100 mM Tris-HCl buffer (pH 8) containing 50 mM glyoxal, and the mixture was shaken at 28°C for 6 to 12 hours. Thereafter, the reaction solution was centrifuged, and the obtained supernatant was analyzed by high performance liquid chromatography, thereby confirming and quantifying generation of glyoxylic acid.

When oxidization of glyoxal is catalyzed by the aforementioned oxidase, not only glyoxylic acid but also hydrogen peroxide is produced. Thus, oxidase-producing strains can be found by detecting hydrogen peroxide produced during the reaction. That is to say, 0.1 ml of cell suspension obtained by culture in the aforementioned S medium was added to 0.1 ml of 100 mM phosphate buffer containing 50 mM glyoxal, 1.34 mM 4-AA, 2.18 mM TOOS, and 4 U/ml peroxidase. The obtained mixture was shaken at 28°C for 2 hours. Thereafter, reaction solutions, the color of which became violet, namely, strains generating hydrogen peroxide as a result of the reaction with glyoxal, were selected, so as to obtain strains having glyoxal oxidase activity.

Examples of microorganisms that can convert glyoxal into glyoxylic acid may include those belonging to the genus Stenotrophomonas, Streptomyces, Pseudomonas, Microbacterium, Achromobacter, Cellulomonas, Cellulosimicrobium, and Morganella. Of these, examples of typical microorganisms may include *Stenotrophomonas sp.* KNK235 (FERM P-19002), *Streptomyces sp.* KNK269 (FERM BP-08556), *Pseudomonas sp.* KNK058 (FERM BP-08555), *Pseudomonas sp.* KNK254 (FERM P-19003), *Microbacterium sp.* KNK011 (FERM BP-08554), *Achromobacter sp*. IFO 13495, *Cellulomonas sp.* JCM 2471, *Cellulomonas turbata* IFO 15012, *Cellulomonas turbata* IFO 15014, *Cellulomonas turbata* IFO 15015, *Cellulosimicrobium cellulans* IFO 15013, *Cellulosimicrobium cellulans* IFO 15516, *Cellulosimicrobium cellulans* JCM 6201, and *Morganella morganii* IFO 3848. Of these microorganisms, IFO 13495, IFO 15012, IFO 15014, IFO 15015, IFO 15013, IFO 15516, and IFO 3848 have already been known. These microorganisms are easily available from National Institute of Technology and Evaluation, Biological Resource Center (NBRC), (Kazusa Kamatari 2-5-8, Kisarazu, Chiba, Japan (postal code: 292-0818)). JCM 2471 and JCM 6201 have also already been known. These microorganisms are easily available from Riken Bioresource Center, Japan Collection of Microorganisms (JCM) (Hirosawa 2-1, Wako, Saitama, Japan (postal code: 351-0198)). Other microorganisms were newly separated from the soil and identified by the present inventors. The thus identified microorganisms were then independently deposited with National Institute of Advanced Industrial Science and Technology (AIST Tsukuba, Central 6, Higashi 1-1-1, Tsukuba, Ibaraki, Japan (postal code: 305-8566)) under accession numbers as described above. The mycological properties of the aforementioned *Stenotrophomonas sp.* KNK235 (hereinafter simply referred to as KNK235 at times), *Pseudomonas sp.* KNK058 (hereinafter simply referred to as KNK058 at times), *Pseudomonas sp.* KNK254 (hereinafter simply referred to as KNK254 at times), and *Microbacterium sp.* KNK011 (hereinafter simply referred to as KNK011 at times) are shown in Table 1.

**Table 1**

| | KNK235 | KNK254 | KNK058 | KNK011 |
|---|---|---|---|---|
| Form of cells | Bacillus (0.8 x 2.0 to 3.0 µm) | Bacillus (0.7 to 0.8 x 2.0 to 2.5 µm) | Bacillus (0.8 x 1.5 to 2.0 µm) | Bacillus (0.7 to 0.8 x 1.0 to 1.2 µm) |
| Gram staining | - | - | - | + |
| Spore formation | - | - | - | - |
| Mobility | + Round shape, smooth entire fringe, small | + Round shape, smooth entire fringe, small | + Round shape, smooth entire fringe, small | - Round shape, smooth entire fringe, small |
| Form of colony | degree of convex, lustrous, yellow | degree of convex, lustrous, yellow | degree of convex, lustrous, yellow | degree of convex, lustrous, yellow |
| Culture temperature | +(37°C) -(45°C) | - (37°C) - (45°C) | +(37°C) -(45°C) | +(37°C) -(45°C) |
| Catalase | + | + | + | + |
| Oxidase | - | + | - | - |
| OF test (glucose) | - | - | - | - |
| Nitrate reduction | - | - | - | - |
| Pyrazinamidase | | | | + |
| Pyrrolidonyl allyl amidase | | | | - |
| β-glucuronidase | | | | - |
| β-galactosidase | + | + | | + |
| α-glucosidase | | | | + |
| N-acetyl-β-glucosaminidase | | | | - |
| Esculin (β-glucosidase) | | + | | + |
| Arginine dihydrase | - | - | - | |
| Cytochrome oxidase | - | + | - | |
| Urease | - | - | - | - |
| Gelatin hydrolysis | + | + | + | + |
| Generation of indole | - | - | - | |
| Fermentability | | | | |
| Glucose | | + | + | + |
| Ribose | | | | - |
| Xylose | | | | + |
| Mannitol | | - | | + |
| Maltose | + | + | - | + |
| D-mannose | + | + | + | |
| L-arabinose | - | - | + | |
| D-mannitol | - | - | + | |
| N-acetyl-D- | + | - | - | |
| glucosamine | | | | |
| Potassium gluconate | - | - | + | |
| n-capric acid | - | - | + | |
| Adipic acid | - | - | - | |
| Malic acid | | + | + | |
| Sodium citrate | + | + | + | |
| Phenyl acetate | - | - | - | |
| Lactose | | | | - |
| Saccharose | | | | + |
| Glycogen | | | | - |

*Streptomyces sp.* KNK269 (hereinafter simply referred to as KNK269 at times) has been identified by the following publicly known method. An approximately 500-bp region on the 5'-terminal side of a 16S ribosomal RNA gene (16Sr DNA) of the above strain was amplified by PCR, and the nucleotide sequence thereof was then determined. Thereafter, homologous search was carried out by a method of producing a molecular cladogram, using MicroSeq Bacterial 500 library v. 0023 database (Applied Biosystems, CA, U.S.A.).

When the aforementioned aldehyde oxidase is produced by microorganisms belonging to the genus Streptomyces, the aldehyde oxidase preferably has the following physicochemical properties (1) to (3):
(1) optimum pH: 6 to 9;
(2) heat stability: the aldehyde oxidase retains activity of 90% or more after it has been treated at pH 7.2 at 60°C for 20 minutes; and
(3) molecular weight: the aldehyde oxidase has a molecular weight of approximately 110,000 in gel filtration analysis, and has three subunit proteins with molecular weights of approximately 25,000, approximately 35,000, and approximately 80,000 in SDS-polyacrylamide gel electrophoresis analysis.

Among microorganisms belonging to the genus Streptomyces, *Streptomyces sp.* KNK269 (FERM BP-08556) is preferable.

When the aforementioned aldehyde oxidase is produced by microorganisms belonging to the genus Pseudomonas, the aldehyde oxidase preferably has the following physicochemical properties (1) to (3):
(1) molecular weight: approximately 150,000 in gel filtration analysis;
(2) optimum reaction temperature: 60°C to 70°C; and
(3) optimum reaction pH: 5 to 7.

Among microorganisms belonging to the genus Pseudomonas, *Pseudomonas sp.* KNK058 (FERM BP-08555) is preferable.

When the aforementioned aldehyde oxidase is produced by microorganisms belonging to the genus Microbacterium inside and outside of the cells thereof, the aldehyde oxidase preferably has the following physicochemical properties:
molecular weight: a single protein has a molecular weight of approximately 110,000 in SDS-polyacrylamide gel electrophoresis analysis.

Among microorganisms belonging to the genus Microbacterium, *Microbacterium sp*. KNK011 (FERM BP-08554) is preferable.

When the aforementioned aldehyde oxidase is produced by microorganisms belonging to the genus Cellulosimicrobium inside and outside of the cells thereof, the aldehyde oxidase preferably has the following physicochemical properties:
molecular weight: a single protein has a molecular weight of approximately 90,000 to 100,000 in SDS-polyacrylamide gel electrophoresis analysis.

Among microorganisms belonging to the genus Cellulosimicrobium, *Cellulosimicrobium cellulans* IFO 15516 is preferable.

In the present invention, a medium used for culturing microorganisms that can produce oxidoreductase that can convert glyoxal into glyoxylic acid is not particularly limited, as long as the above microorganisms can proliferate therein. An example of such a medium used herein may be a common liquid medium, which comprises: carbon sources including sugars such as glucose or sucrose, alcohols such as ethanol, glycerol, ethylene glycol, or propylene glycol, aldehydes such as glyoxal, fatty acids such as oleic acid or stearic acid and the esters thereof, and oils such as rapeseed oil or soybean oil; nitrogen sources such as ammonium sulfate, sodium nitrate, peptone, casamino acid, yeast extract, meat extract, or corn steep liquor; inorganic salts such as magnesium sulfate, sodium chloride, calcium carbonate, dipotassium hydrogen phosphate, or potassium dihydrogen phosphate; and other components such as malt extract or meat extract.

The process for producing glycolic acid of the present invention is characterized in that it comprises allowing any one selected from the group consisting of the aforementioned oxidoreductase, a culture broth containing a microorganism that can produce the above described oxidoreductase, cells separated from the culture broth, a processed product thereof, and a supernatant of a culture broth in a case where the above oxidase is produced even outside of a microorganism , to react with glyoxal, so as to convert it into glyoxylic acid and accumulate it.

Herein, the term " processed product of microorganism " is used to mean a freeze-dried cells, an acetone-dried cells, a disrupted product of such cells, a crude enzyme solution, or the like. The term "crude enzyme solution" includes a solution obtained by disrupting or lysing cells by physical disruption methods using glass beads or the like or by biochemical methods using enzymes or the like, a cell-free extract obtained by removing solids from the above solution by centrifugation or the like, and so on. Moreover, the term "crude enzyme solution" further includes an enzyme obtained by partially purifying the aforementioned cell-free extract, using dialysis, ammonium sulfate precipitation, or chromatography, singly or in combination. Furthermore, such a processed product of microorganism may be immobilized by known means, before use. Such immobilization can be carried out by methods publicly known to persons skilled in the art (for example, crosslinking method, physical absorption method, encapsulation method, etc.).

Reaction conditions are different depending on an enzyme used, a microorganism used, or a processed product thereof. Optimum reaction conditions are as follows. The temperature is between 10°C and 80°C, and preferably between 20°C and 40°C from the viewpoint of heat stability. The pH is between pH 4 and 12, and preferably between pH 6 and 10 from the viewpoint of pH stability. The reaction is preferably carried out under conditions consisting of shaking and agitation.

When the reaction is catalyzed by oxidase, hydrogen peroxide is generated in the reaction system. Hydrogen peroxide may inactivate enzymes or may decompose glyoxylic acid into formic acid. Such hydrogen peroxide generated as a result of the reaction can be decomposed and removed by addition of catalase, so as to prevent inactivation of enzymes or decomposition of glyoxylic acid.

As an enzyme of the present invention, an enzyme exhibiting only low activity to glyoxylic acid is desirable. In particular, the activity of the enzyme of the present invention to glyoxylic acid is preferably one-tenth of or less than, more preferably one-twentieth of or less than, and further preferably one-hundredth of or less than the activity thereof to glyoxal. If the activity of oxidase to glyoxylic acid exceeds one-tenth of the activity thereof to glyoxal, glyoxal is oxidized, and the generated glyoxylic acid is further oxidized. As a result, it is likely that glyoxylic acid is not accumulated in the reaction system or that the amount of glyoxylic acid accumulated is decreased. Thus, the oxidase of the present invention is characterized in that it does not only convert glyoxal into glyoxylic acid, but also its activity to glyoxylic acid is low. Glyoxal oxidase generated by wood-rotting fungi, which reportedly exhibit activity to glyoxal, exhibits high activity also to glyoxylic acid. Table 2 shows the activities of the enzymes of the present invention and enzyme generated by wood-rotting fungi to glyoxal and to glyoxylic acid. When compared with that activity to glyoxal, the enzymes of the present invention exhibit extremely low activity to glyoxylic acid.

**Table 2**

| Substrate | Relative activity (%) | | | | | |
|---|---|---|---|---|---|---|
| | P. chrysosporium | KNK 235 | KNK 269 | KNK 058 | KNK 011 | IFO 15516 |
| Glyoxal | 100 | 100 | 100 | 100 | 100 | 100 |
| Glyoxylic acid | 60 | 5 | 0.1 | 0.1 | 0.5 | 1 |

Moreover, the present invention relates to DNA encoding the aforementioned aldehyde oxidase, which can be used for efficient production of the aforementioned aldehyde oxidase using genetic recombination. Specifically, the present invention relates to DNA encoding the subunit of aldehyde oxidase, which has the nucleotide sequence represented by SEQ ID NOS: 4, 5, or 6. Further, DNA hybridizing with a DNA having a nucleotide sequences that is complementary to the DNA having the above nucleotide sequence under stringent conditions is also included in the DNA of the present invention, as long as a protein having a protein encoded by the above DNA as a subunit has activity of converting glyoxal into glyoxylic acid.

Furthermore, the present invention also relates to DNA encoding aldehyde oxidase, which has the nucleotide sequence represented by SEQ ID NO: 9, 10, 13, or 14. Further, DNA hybridizing with a DNA consisting of a nucleotide sequence that is complementary to the DNA consisting of the above nucleotide sequence under stringent conditions is also included in the DNA of the present invention, as long as a protein encoded by the above DNA has activity of converting glyoxal into glyoxylic acid.

Still further, DNA encoding a protein comprising any amino acid sequence resulting from deletion, substitution, or addition of one or several amino acids in the amino acid sequence represented by SEQ ID NO: 1, 2, or 3, is also included in the DNA of the present invention, as long as a protein having the protein encoded by the above DNA as a subunit has activity of converting glyoxal into glyoxylic acid.

Still further, DNA encoding a protein comprising an amino acid sequence resulting from deletion, substitution, or addition of one or several amino acids in the amino acid sequence represented by SEQ. ID NO: 7, 8, 11, or 12, is also included in the DNA of the present invention, as long as the protein encoded by the above DNA has activity of converting glyoxal into glyoxylic acid.

The present invention also relates to aldehyde oxidase having a protein encoded by DNA having the nucleotide sequence represented by SEQ ID NO: 4, 5, or 6 as a subunit, and to aldehyde oxidase encoded by DNA having the nucleotide sequence represented by SEQ ID NO: 9, 10, 13, or 14. Moreover, aldehyde oxidase that has, as a subunit, a protein encoded by DNA hybridizing with any one DNA consisting of a nucleotide sequence that is complementary to the DNA consisting of the nucleotide sequence represented by SEQ ID NOS: 4, 5, or 6, under stringent conditions, and aldehyde oxidase encoded by DNA hybridizing with any one DNA consisting of a nucleotide sequence that is complementary to the DNA consisting of the nucleotide sequence represented by SEQ ID NOS: 9, 10, 13, or 14, under stringent conditions, are also included in the oxidase of the present invention, as long as they have activity of converting glyoxal into glyoxylic acid.

Hybridization can be carried out by operations that have publicly been known to persons skilled in the art. Specifically, hybridization can be carried out by Southern hybridization, using, as probe DNA, DNA obtained by end-labeling double-stranded DNA having the nucleotide sequence represented by SEQ ID NO: 4, 5, 6, 9, 10, 13, or 14, with ³²P according to the nick translation method, for example (refer to Molecular Cloning, 3^{rd} edition, 2001, Cold Spring Harbor Laboratory Press, Cold Spring Harbor, New York, U.S.A., Vol. 1, Chapter 6, 50-55). Chromosomal DNA, genomic DNA, or plasmid DNA, which is produced from any given organism or microorganism, artificially produced vector DNA, or a DNA fragment obtained by digesting the above DNAs with appropriate restriction enzymes, is isolated by agarose gel electrophoresis. Thereafter, the isolated DNA is immobilized onto a nitrocellulose filter, and DNA binding to the above described probe DNA is then detected by autoradiography, thereby detecting a gene of aldehyde oxidase that can be used in the present invention. Stringent conditions applied to the hybridization mean that the DNA immobilized onto the nitrocellulose filter is allowed to hybridize with the labeled probe DNA at 68°C in a buffer consisting of 6 x SSC, 5 x Denhart's reagent, 0.5% SDS, 1µg/m poly (A), and 100 µg/ml salmon sperm DNA, and then that the resultant is rinsed with a buffer consisting of 2 x SSC and 0.5% SDS, followed by washing twice with a buffer consisting of 2 x SSC and 0.1% SDS at 30°C for 30 minutes. More stringent conditions include the case where the above washing is carried out 4 times with a buffer consisting of 1 x SSC and 0.5% SDS at 65°C for 30 minutes. 1 x SSC is an aqueous solution containing 0.15 M sodium chloride and 0.015 M sodium citrate. 1 x Denhart's reagent contains 0.02% Ficoll 400 (manufactured by Sigma-Aldrich Corporation), 0.02% polyvinylpyrrolidone, and 0.02% bovine serum albumin (manufactured by Sigma-Aldrich Corporation; Fraction V).

Furthermore, the present invention also relates to aldehyde oxidase having a protein consisting of the amino acid sequence represented by SEQ ID NO: 1, 2, or 3 as a subunit, and aldehyde oxidase having the amino acid sequence represented by SEQ ID NO: 7, 8, 11, or 12. Still further, a protein having an amino acid sequence resulting from deletion, substitution, or addition of one or several amino acids in any one of the amino acid sequences of the subunits γ, β, and α of aldehyde oxidase represented by SEQ ID NOS: 1, 2, and 3, is also included in the oxidase of the present invention, as long as such a protein having the above protein as a subunit has activity of converting glyoxal into glyoxylic acid. Still further, a protein having an amino acid sequence resulting from deletion, substitution, or addition of one or several amino acids in any one of the amino acid sequences represented by SEQ ID NOS: 7, 8, 11, and 12, is also included in the oxidase of the present invention, as long as it has activity of converting glyoxal into glyoxylic acid.

As a method of deletion, substitution, or addition of a specific amino acid(s), a conventionally known method is used. Examples of such a method may include: PCR using synthetic DNA primers having nucleotide sequences comprising deletion of codons of specific amino acids, substitution with codons of other amino acids, or addition of codons of other amino acids; and a method of ligating an enzyme gene produced by known methods such as a chemical DNA synthesis method to a gene expression vector, and allowing it to express in a host such as *Escherichia coli* by genetic recombination. These methods can easily be carried out by persons skilled in the art.

In the case of an enzyme secreted outside of the cells of microorganism, a secretory signal sequence is generally encoded in a portion corresponding to several tens of amino acids from the initiation codon of the gene. It has been known that such a secretory signal sequence is cleaved so as to become a mature enzyme during a step in which an enzyme protein synthesized in the cells is secreted to outside of the cells. In the case of several enzymes described in the present invention, the active enzymes are secreted also to outside of the cells. In the production of these enzymes by genetic recombination, an enzyme protein can be produced inside of the cells of a microorganism used as a host, using a gene from which a secretory signal sequence has artificially been removed. The present invention also provides enzyme genes (SEQ ID NOS: 10 and 14), from which secretory signal sequences have been removed and which can be used for the above described purpose. On the other hand, for the purpose of producing an enzyme of the cells of a host microorganism in the expression of the enzyme by genetic recombination, genes comprising secretory signal sequences (SEQ ID NOS: 9 and 13) can also be used. Further, a chimeric gene, the original secretory signal sequence of which has been substituted with another secretory signal sequence that is suitable for a host microorganism, can be constructed and used for the above described purpose by persons skilled in the art according to known methods.

The gene of an enzyme usable in the present invention can be obtained by the following method. That is to say, an enzyme protein is purified from a microorganism that produces an enzyme converting glyoxal into glyoxylic acid, or from a culture broth thereof. Thereafter, using a peptide obtained by digesting the enzyme protein with protease, partial amino acid sequences are determined. Subsequently, using primers synthesized based on these partial amino acid sequences, PCR is carried out with genomic DNA as a template according to publicly known methods. By such PCR, a part of the enzyme gene is amplified, and the nucleotide sequence of the inside of the gene can be determined. Also, inverse PCR is carried out using DNA primers synthesized from an N-terminal amino acid sequence and an amino acid sequence around the C-terminus, so as to determine a signal sequence, an N-terminal amino acid sequence, a C-terminal amino acid sequence, etc. (Cell Science 1990, vol. 6, No. 5, 370-376). The gene of aldehyde oxidase, the nucleotide sequence of which has been determined, can easily be obtained by PCR. Moreover, it is also possible to obtain such an aldehyde oxidase gene from the chromosomal DNA or genomic DNA of a microorganism by known methods using a partial sequence of the gene.

An enzyme used in the present invention may be either a natural enzyme or an enzyme obtained by recombinant technology. As such recombinant technology, a method of inserting an enzyme gene into a plasmid vector or a phage vector, and transforming a host including bacteria such as *Escherichia coli*, microorganisms such as yeast or fungi, or cells of such animals or plants, with the above vector, is effective, for example.

The present invention will be described more in detail in the following examples. However, these examples are not intended to limit the present invention.

### (Example 1)

5 ml of liquid medium (EG-NB medium (pH 7)) with the composition consisting of 10 g of ethylene glycol, 1 g of yeast extract, 8 g of NUTRIENT BROTH (manufactured by Difco), 3 g of potassium dihydrogen phosphate, and 7 g of dipotassium hydrogen phosphate (all of which were amounts contained in 1 liter), was poured into a large test tube, and it was then sterilized by autoclaving at 121°C for 20 minutes. Using an inoculating loop, each of the microorganisms shown in Table 3 was aseptically inoculated into this medium. It was then cultured at 28°C for 2 days, so as to obtain a preculture broth. Subsequently, 1 ml of the obtained preculture broth was inoculated into 100 ml of the sterilized EG-NB medium placed in a 500-ml Sakaguchiflask, and it was then cultured at 28°C for 3 days. The cells were collected from 100 ml of the obtained culture broth by centrifugation. The cells were washed with a 100 mM Tris-HCl buffer (pH 8.0) and were then suspended in 5 ml of the same above buffer (pH 8.0). The cell suspension was disrupted with Mini Beat-Beater (manufactured by BIOSPEC) and followed by centrifugation, so as to obtain a supernatant (a cell-free extract). 0.1 ml of a 500 mM glyoxal aqueous solution and 0.1 ml of a solution containing 50,000 U/ml catalase were added to 0.8 ml of the obtained cell-free extract, and the obtained mixture was subjected to a shaking reaction in a test tube at 28°C for 4 hours. The obtained reaction solution was analyzed by high performance liquid chromatography. The amount of glyoxylic acid produced is summarized in Table 3.

**Table 3**

| Strain | Amount of glyoxylic acid produced (mM) |
|---|---|
| *Stenotrophomonas sp.* KNK235 | 2 |
| *Streptomyces sp.* KNK269 | 5 |
| *Pseudomonas sp.* KNK254 | 10 |
| *Pseudomonas sp.* KNK 058 | 12 |
| *Microbacterium sp.* KNK011 | 24 |
| *Achromobacter sp.* IFO 13495 | 2 |
| *Cellulomonas sp.* JCM 2471 | 32 |
| *Cellulomonas turbata* IFO 15012 | 13 |
| *Cellulomonas turbata* IFO 15014 | 15 |
| *Cellulomonas turbata* IFO 15015 | 20 |
| *Cellulosimicrobium cellulans* IFO 15013 | 14 |
| *Cellulosimicrobium cellulans* IFO 15516 | 23 |
| *Cellulosimicrobium cellulans* JCM 6201 | 21 |
| *Morganella morganii* IFO 3848 | 7 |

### (Example 2)

0.05 ml of 0.1 M phosphate buffer (pH 7) containing 1.34 mM 4-AA, 2.19 mM TOOS, and 6 U/ml POD was added to 0.1 ml of the cell-free extract of each of the microorganisms shown in Table 3, which had been prepared in Example 1, in a test tube. Thereafter, 0.05 ml of 100 mM glyoxal aqueous solution or water was added thereto, and the mixture was shaken at 28°C for 2 minutes. Thereafter, a change in the color of the reaction solution was observed. The results are shown in Table 4. In all the reactions of using any one of the above-described microorganisms, the reaction solution obtained when a glyoxal aqueous solution had been added exhibited a strong violet color. When water was added instead of such a glyoxal aqueous solution, the reaction solution did not change color. It was found that hydrogen peroxide is generated during the oxidization reaction of glyoxal. From this fact, it was found that an enzyme catalyzing the oxidization reaction of glyoxal is oxidase.

**Table 4**

| Strain | Coloration of reaction solution | |
|---|---|---|
| | Glyoxal added | Glyoxal not added |
| *Stenotrophomonas sp.* KNK 235 | + | - |
| *Streptomyces sp.* KNK 269 | + - | - |
| *Pseudomonas sp*. KNK 254 | + - | - |
| *Pseudomonas sp*. KNK 058 | + - | - |
| *Microbacterium sp.* KNK011 | + - | - |
| *Achromobacter sp.* IFO 13495 | + | - |
| *Cellulomonas sp.* JCM 2471 | + - | - |
| *Cellulomonas turbata* IFO 15012 | + | - |
| *Cellulomonas turbata* IFO 15014 | + | - |
| *Cellulomonas turbata* IFO 15015 | + | - |
| *Cellulosimicrobium cellulans* IFO 15013 | + | - |
| *Cellulosimicrobium cellulans* IFO 15516 | + | - |
| *Cellulosimicrobium cellulans* JCM 6201 | + | - |
| *Morganella morganii* IFO 3848 | + | - |

### (Example 3)

A culture broth of each of *Pseudomonas sp.* KNK254, *Microbacterium sp.* KNK 011, *Cellulomonas turbata* IFO 15015, and *Cellulomonas sp.* JCM2471 was prepared by the same method as described in Example 1. Thereafter, the cells were collected from 100 ml of the obtained culture broth by centrifugation, and were then washed with a 0.1 mM phosphate buffer (pH 7). Thereafter, the cells were suspended in 5 ml of the same above buffer. Thereafter, 0.05 ml of 500 mM glyoxal aqueous solution was added to 0.45 ml of the present cell suspension placed in a test tube, and the obtained mixture was reacted by shaking for 4 hours. After completion of the reaction, the obtained supernatant was analyzed by HPLC, and the amount of glyoxylic acid produced was calculated. As a result, it was found that 20 mM glyoxylic acid was produced from *Pseudomonas sp.* KNK 254, 14 mM glyoxylic acid was produced from *Microbacterium sp.* KNK011, 30 mM glyoxylic acid was produced from *Cellulomonas turbata* IFO 15015, and 33 mM glyoxylic acid was produced from *Cellulomonas sp.* JCM 2471.

### (Example 4)

Aldehyde oxidase having activity of converting glyoxal into glyoxylic acid was purified from *Streptomyces sp.* KNK 269 by the following method.

50 ml of a medium (pH 7) with the composition consisting of 10 g of ethylene glycol, 3 g of yeast extract, 8 g of Nutrient Broth, 3 g of dipotassium hydrogen phosphate, and 7 g of dipotassium hydrogen phosphate (all of which were amounts contained in 1 liter), was poured into a 500-ml Sakaguchi flask, and it was then sterilized by autoclaving. Using an inoculating loop, *Streptomyces sp.* KNK 269 was inoculated into this medium. It was then subjected to shake culture at 28°C for 3 days, so as to obtain a preculture broth. Subsequently, 6 L of the medium with the above composition was placed in a 10-L mini jar, and it was then sterilized by autoclaving. Thereafter, 50 ml of the obtained preculture broth was inoculated into the above medium, and it was then cultured at 28°C at aeration of 0.5 vvm at agitation of 300 rpm for 2 days. This mini jar culture was repeated, so as to obtain 95 L of a culture broth. Subsequently, the cells were collected from 95 L of the obtained culture broth by centrifugation, and they were then suspended in 3 L of a 0.05 M phosphate buffer (pH 7).

The obtained cell suspension was disrupted with Dyno Mill (manufactured by Dyno-Mill), and cell residues were then removed by centrifugation, so as to obtain 2.5 L of a cell-free extract. While stirring with a stirrer under cooling on ice, a predetermined amount of ammonium sulfate was added to 2.5 L of the obtained cell-free extract. Thereafter, proteins precipitated with 30% to 55% saturation of ammonium sulfate were collected by centrifugation.

The obtained proteins were dissolved in a 0.05 M phosphate buffer (pH 7), and dialysis was carried out with the same buffer. The resultant solution was charged to a DEAE-TOYOPEARL 650 M column (manufactured by Tosoh Corporation) (130 ml) that had previously been equilibrated with the same buffer, and fractions that passed by the column were eliminated. Thereafter, the remaining protein was eluted with a 0.05 M phosphate buffer (pH 7) containing 0.5 M sodium chloride, so as to collect active fractions. Thereafter, ammonium sulfate was added to the obtained enzyme solution such that the concentration of ammonium sulfate became 0.6 M. The mixed solution was charged to a Phenyl-TOYOPEARL 650 M column (manufactured by Tosoh Corporation) (300 ml) that had previously been equilibrated with a 0.05 M phosphate buffer containing 0.6 M ammonium sulfate. Thereafter, the enzyme was eluted with a linear concentration gradient of ammonium sulfate from 0.6 to 0.1 M, so as to collect active fractions. The obtained enzyme solution was dialyzed with a 0.05 M phosphate buffer (pH 7) . The resultant solution was charged to a DEAE-TOYOPEARL 650 M column (130 ml) that had previously been equilibrated with the same above buffer. Thereafter, the enzyme was eluted with a linear concentration gradient of sodium chloride from 0 to 0.25 M, so as to collect active fractions. Thereafter, ammonium sulfate was added thereto until it became 60% saturated. Precipitated proteins were collected by centrifugation and were then dissolved in a 0.05 M phosphate buffer (pH 7), followed by dialysis with the same buffer. Subsequently, the enzyme solution obtained after the dialysis was charged to a Benzamidine Sepharose column (manufactured by Amersham Pharmacia Biotech) (10 ml) that had previously been equilibrated with a 0.05 M phosphate buffer (pH 7). Thereafter, the enzyme was eluted with a linear concentration gradient of sodium chloride from 0 to 0.1 M, so as to collect active fractions. This enzyme solution was concentrated by ultrafiltration. The concentrate was then charged to a Superdex 200HR 16/60 column (manufactured by Amersham Pharmacia Biotech) (120 ml) that had previously been equilibrated with a 0. 05 M phosphate buffer (pH 7) containing 0.15 M sodium chloride. Thereafter, the solution was eluted with the same above buffer. An elution peak with the same activity as that of protein absorption at 280 nm was obtained from a fraction corresponding to a molecular weight of 170,000. When this active fraction was subjected to native polyacrylamide gel electrophoresis, it formed a single band.

On the other hand, when the present enzyme was subjected to SDS-polyacrylamide gel electrophoresis, it formed three protein bands corresponding to molecular weights of approximately 25,000, 35,000, and 80,000. From these results, it was found that the present enzyme has a structure consisting of subunits with molecular weights of approximately 25,000, 35,000, and 80,000.

### (Example 5)

Aldehyde oxidase having activity of converting glyoxal into glyoxylic acid was purified from *Microbacterium sp.* KNK 011 by the following method.

50 ml of medium (pH 7) with the composition consisting of 5 g of yeast extract, 2 g of ammonium nitrate, 2 g of dipotassium hydrogen phosphate, 1 g of sodium dihydrogen phosphate dihydrate, 0.2 g of magnesium sulfate heptahydrate, and 0.1 g of calcium chloride dihydrate (all of which were amounts contained in 1 liter), was poured into a 500-ml flask, and it was then sterilized by autoclaving. Using an inoculating loop, *Microbacterium sp.* KNK 011 was inoculated into this medium. It was then subjected to shake culture at 28°C for 3 days, so as to obtain a preculture broth. Subsequently, 3 L of the medium with the above composition was placed in a 5-L mini jar, and it was then sterilized by autoclaving. Thereafter, 30 ml of the obtained preculture broth was inoculated into the above medium, and it was then cultured at 28°C at aeration of 0.5 vvm at agitation of 400 rpm for 28 hours. This mini jar culture was repeated, so as to obtain 69 L of a culture broth. Subsequently, the cells were collected from 69 L of the obtained culture broth by centrifugation, and they were then suspended in 0.05 M phosphate buffer (pH 7). The obtained cells suspension was disrupted with Dyno Mill (manufactured by Dyno-Mill), and cell residues were then removed by centrifugation, so as to obtain 2 L of a cell-free extract. While stirring with a stirrer under cooling on ice, a predetermined amount of ammonium sulfate was added to 2 L of the obtained cell-free extract. Thereafter, proteins precipitated with 20% to 40% saturation of ammonium sulfate were collected by centrifugation.

The obtained proteins were dissolved in a 0.05 M phosphate buffer (pH 7), and dialysis was carried out with the same buffer. The resultant solution was charged to a DEAE-TOYOPEARL 650 M column (300 ml) that had previously been equilibrated with the same buffer. Thereafter, the enzyme was eluted with a linear concentration gradient of sodium chloride from 0 to 0.6 M, so as to collect active fractions. Thereafter, a predetermined amount of ammonium sulfate was added to the obtained active fractions such that the concentration of ammonium sulfate became 0.7 M. The mixed solution was charged to a Phenyl-TOYOPEARL 650 M column (160 ml) that had previously been equilibrated with a 0.05 M phosphate buffer (pH 7) containing 0.7 M ammonium sulfate. Thereafter, the enzyme was eluted with a linear concentration gradient of ammonium sulfate from 0.7 to 0 M, so as to collect active fractions. The obtained enzyme solution was dialyzed with a 0.05 M phosphate buffer (pH 7). Thereafter, the enzyme solution obtained after the dialysis was charged to a Resource Q column (manufactured by Amersham Pharmacia Biotech) (6 ml) that had previously been equilibrated with a 0.05 M phosphate buffer. Thereafter, the solution was eluted with linear concentration gradient of sodium chloride from 0.15 to 0.45 M, so as to collect active fractions. The obtained enzyme solution was concentrated by ultrafiltration. Thereafter, ammonium sulfate was added thereto such that the concentration thereof became 0.3 M. The obtained mixed solution was charged to a Resource Phe column (manufactured by Amersham Pharmacia Biotech) (6 ml) that had previously been equilibrated with a 0.05 M phosphate buffer (pH 7) containing 0.3 M ammonium sulfate. Thereafter, the enzyme was eluted with a linear concentration gradient of ammonium sulfate from 0.3 to 0 M, so as to collect active fractions.

The obtained enzyme solution was subjected to SDS-polyacrylamide gel electrophoresis. As a result, it formed a single protein band corresponding to a molecular weight of approximately 110,000.

### (Example 6)

Aldehyde oxidase having activity of converting glyoxal into glyoxylic acid was purified from *Pseudomonas sp.* KNK058 by the following method.

5 ml of medium (pH 7) with the composition consisting of 10 g of ethylene glycol, 8 g of nutrient broth, 7 g of dipotassium hydrogen phosphate, and 3 g of potassium dihydrogen phosphate (all of which were amounts contained in 1 liter), was sterilized by autoclaving in a large test tube. Using an inoculating loop, *Pseudomonas sp.* KNK058 was inoculated into this medium, and it was cultured at 28°C for 2 days. Subsequently, the obtained culture broth was inoculated into 500 ml of the above-sterilized medium placed in a 2-L shake flask, and thus, it was subjected to shake culture at 28°C for 18 hours, so as to obtain a preculture broth. Subsequently, the obtained preculture broth was inoculated into 60 L of the above-sterilized medium placed in a jar fermenter, and it was then cultured at 28°C at aeration of 1 vvm at agitation of 200 rpm for 40 hours. Thereafter, cells were collected from 60 L of the obtained culture broth by centrifugation, and they were then suspended in a 0.02 M phosphate buffer (pH 7). The obtained cells suspension was disrupted with an Inconator 201M ultrasonic disintegration device (manufactured by Kubota Corporation) for 60 minutes, and cell residues were then removed by centrifugation, so as to obtain a cell-free extract. While the obtained cell-free extract was stirred with a stirrer under cooling, a predetermined amount of ammonium sulfate was added thereto. Thereafter, proteins precipitated with 20% to 60% saturation of ammonium sulfate were collected by centrifugation.

The obtained proteins were dissolved in a 0.02 M phosphate buffer (pH 7), and dialysis was carried out with the same buffer. Thereafter, 1.5 L of DEAE-Sephacel resin was added to the enzyme solution, and the mixture was stirred at 4°C for 1 hour. Thereafter, an unadsorbed protein solution was removed by filtration, and an enzyme protein adsorbed onto the resin was eluted with 1 M sodium chloride.

The obtained enzyme solution was dialyzed with a 0.02 M phosphate buffer (pH 7), and it was then charged to a HiPrep 16/10-Q-XL column (manufactured by Amersham Pharmacia Bioscience) (16 ml) that had previously been equilibrated with a 0.02 M phosphate buffer (pH 7), and elution was carried out with a linear concentration gradient of sodium chloride from 0 to 1 M, so as to collect active fractions. A predetermined amount of ammonium sulfate was added to the active fractions such that the concentration thereof became 1.2 M. The mixed solution was charged to a Phenyl Superose HR 10/10 column (manufactured by Amersham Pharmacia Bioscience) (10 ml) that had previously been equilibrated with a 0.02 M phosphate buffer containing 1.2 M ammonium sulfate. Thereafter, the enzyme was eluted with a linear concentration gradient of ammonium sulfate from 1.2 to 0 M, so as to collect active fractions. Thereafter, the obtained enzyme solution was dialyzed with a 0.02 M phosphate buffer. The resultant solution was charged to a MonoQ HR 10/10 column (manufactured by Amersham Pharmacia Bioscience) (10 ml) that had previously been equilibrated with the same above buffer. Thereafter, the solution was eluted with linear concentration gradient of sodium chloride, so as to collect active fractions. Also, the obtained enzyme solution was charged to a HiPrep Sephacryl S-200 16/60 column (manufactured by Amersham Pharmacia Bioscience) (60 ml) that had previously been equilibrated with a 0.02 M phosphate buffer containing 0.02 M sodium chloride, and the solution was eluted with the same above buffer. Active fractions were collected, and they were then dialyzed with a 0.005 M phosphate buffer (pH 7). The resultant solution was charged to a Hydroxyapatite column (manufactured by Seikagaku Corporation) (10 ml) that had previously been equilibrated with the same above buffer, and the enzyme was eluted with a linear concentration gradient of a phosphate buffer from 0.005 to 0.5 M. Active fractions were collected, and they were then dialyzed with a 0.005 mM phosphate buffer. The resultant solution was charged to a Bio-Scale CHT5-I column (manufactured by Bio-Rad) (6.4 ml) that had previously been equilibrated with the same above buffer, and the enzyme was eluted with a linear concentration gradient of a phosphate buffer from 0.005 to 0.5 M, so as to collect active fractions. When the active fractions were subjected to native polyacrylamide gel electrophoresis, it formed a single band.

### (Example 7)

Aldehyde oxidase having activity of converting glyoxal into glyoxylic acid was purified from the culture supernatant of *Cellulosimicrobium cellulans* IFO 15516 by the following method.

60 ml of medium (pH 7) with the composition consisting of 10 g of yeast extract, 2 g of ammonium sulfate, 1 g of dipotassium hydrogen phosphate, 1 g of sodium dihydrogen phosphate, 0.2 g of magnesium sulfate heptahydrate, and 0.1 g of calcium chloride dihydrate (all of which were amounts contained in 1 liter), was poured into a 500-ml Sakaguchi flask, and it was then sterilized by autoclaving. Thereafter, using an inoculating loop, *Cellulosimicrobium cellulans* NBRC 15516 was inoculated into this medium. It was then subjected to shake culture at 28°C for 2 days, so as to obtain a preculture broth. Subsequently, 3 L of the medium with the above composition was placed in a 5-L mini jar, and it was then treated by autoclaving. Thereafter, 60 ml of the obtained preculture broth was inoculated into the above medium, and it was then cultured at 28°C at aeration of 0.5 vvm at agitation of 400 rpm for 27 hours. The same culture was repeated, so as to obtain 45 L of a culture broth. The obtained culture broth was adjusted to be pH 7, and it was then centrifuged, so as to obtain 45 L of a culture supernatant. The obtained culture supernatant was concentrated to 2.4 L using agitation-type Ultra Holder UHP150 (manufactured by Advantec Toyo). Thereafter, while the concentrate was stirred under cooling on ice, a predetermined amount of ammonium sulfate was added thereto. Thereafter, proteins precipitated with 0% to 60% saturation of ammonium sulfate were collected by centrifugation. The obtained proteins were dissolved in a 20 mM potassium phosphate buffer (pH 7), and dialysis was carried out with a sufficient amount of the same buffer. The resultant solution was charged to a DEAE-TOYOPEARL 650 M column (300 ml) that had previously been equilibrated with the same above buffer. Thereafter, the enzyme was eluted with a linear concentration gradient of sodium chloride from 0 to 0.5 M, so as to collect active fractions. Thereafter, a predetermined amount of ammonium sulfate was added to the obtained active fractions to a final concentration of 1 M. The mixed solution was charged to a Phenyl-TOYOPEARL 650 M column (60 ml) that had previously been equilibrated with a 20 mM potassium phosphate buffer (pH 7) containing 1 M ammonium sulfate. Thereafter, the enzyme was eluted with a linear concentration gradient of ammonium sulfate from 1 to 0.5 M, so as to collect active fractions. The obtained enzyme solution was dialyzed with a 20 mM potassium phosphate buffer (pH 7). Thereafter, the resultant solution was charged to a Resource Q column (manufactured by Amersham Pharmacia Biotech) (6 ml) that had previously been equilibrated with the same above buffer. Thereafter, the solution was washed with the same above buffer containing 0.35 M sodium chloride, and the enzyme was then eluted with a linear concentration gradient of sodium chloride from 0.35 to 0.5 M, so as to collect active fractions. The obtained enzyme solution was concentrated by ultrafiltration. Thereafter, the concentrate was charged to a Superdex 200HR column (manufactured by Amersham Pharmacia Biotech) (24 ml) that had previously been equilibrated with a 20 mM potassium phosphate buffer (pH 7) containing 0.15 M sodium chloride. Thereafter, the solution was eluted with the same above buffer. The obtained active fractions were subjected to SDS-polyacrylamide gel electrophoresis. As a result, it formed a single band with a molecular weight between 90,000 and 100,000.

### (Example 8)

The cell-free extract of each of KNK235 and KNK058 obtained by the method described in Example 1 was charged to a Resource Q column (6 ml) that had previously been equilibrated with a 0.05 M phosphate buffer (pH 7), and the enzyme was eluted with a linear concentration gradient of sodium chloride from 0 to 0.5 M, so as to collect active fractions. Roughly purified enzyme solutions of these KNK235 and KNK058 and purified enzymes obtained from *Streptomyces sp.* KNK269, *Microbacterium sp.* KNK011, and *Cellulosimicrobium cellulans* IFO 15516, obtained in Examples 4, 5, and 7, respectively, were measured in terms of their oxidase activity to glyoxal and to glyoxylic acid. Measurement of the enzyme activity was carried out by the following method. That is, 1.0 ml of a reaction solution comprising 10 mM glyoxal or glyoxylic acid, 0.67 mM4-AA, 1.09 mM TOOS, 2 U/ml POD, and a roughly purified enzyme from KNK235 or KNK058, or a purified enzyme obtained from NKN269, KNK011, or IFO 15516 in Example 4, 5, or 7, was reacted at 30°C in a 100 mM phosphate buffer (pH 7). Thereafter, an increase in the absorbance at a wavelength of 555 nm was measured. A comparison made among the activities of the above enzymes to glyoxal and glyoxylic acid is shown in Table 5.

**Table 5**

| | Activity to glyoxal/activity to glyoxylic acid |
|---|---|
| KNK235-derived enzyme | 19 |
| KNK058-derived enzyme | 1,100 |
| KNK269-derived enzyme | 1,710 |
| KNK011-derived enzyme | 183 |
| IF015516-derived enzyme | 100 |

The activity of each enzyme to glyoxylic acid was lower than that to glyoxal.

### (Example 9)

The physicochemical properties of the enzyme obtained from *Streptomyces sp.* KNK269 in Example 4 were examined. Measurement of the enzyme activity was basically carried out by the method described in Example 8.

### (Optimum pH)

Activity was measured using glyoxal as a substrate within a range between pH 5 and 9. The results are shown in Figure 2. It was found that the optimum pH is between 6 and 9.

### (Heat stability)

The present enzyme was treated in a 0.05 M phosphate buffer (pH 7.2) at each temperature from 30°C to 70°C for 20 minutes. Thereafter, the remaining activity of the present enzyme was measured using glyoxal as a substrate. The results are shown in Figure 3. It was found that 90% or more of the activity remained after the treatment at 70°C.

### (Example 10)

The physicochemical properties of the enzyme obtained from *Pseudomonas sp.* KNK058 in Example 6 were examined. Measurement of the enzyme activity was basically carried out by the method described in Example 8.

### (Molecular weight)

The molecular weight of the present enzyme was measured using TSK-G3000SW column (manufactured by Tosoh Corporation). As a result, it was found to be approximately 150,000.

### (Optimum temperature)

Activity was measured using glyoxal as a substrate within a temperature range between 25°C and 75°C. The results are shown in Figure 4. The enzyme exhibited high activity in a temperature range between 60°C and 70°C.

### (Optimum pH)

Using a McIlvine buffer, a phosphate buffer, or a Tricine buffer as a buffer, activity was measured with glyoxal as a substrate within a pH range between pH 4 and 9. The results are shown in Figure 5. The enzyme exhibited high activity in a pH range between pH 5 and 7.

### (Example 11)

Each of the enzymes derived from *Streptomyces sp.* KNK269, *Microbacterium sp.* KNK011, *Pseudomonas sp.* KNK058, and *Cellulosimicrobium cellulans* IFO 15516, which were obtained in Examples 4, 5, 6, and 7, respectively, was reacted using glyoxal as a substrate. That is, 1 ml of 100 mM Tris-HCl buffer comprising 0.2 U/ml enzyme derived from each of the above strains, 50 mM glyoxal, and 5,000 U/ml catalase, were placed in a test tube. It was then subjected to a shake reaction at 30°C for 3 hours. After completion of the reaction, the reaction solution was analyzed by high performance liquid chromatography. As a result, it was found that 7.6 mM glyoxylic acid was generated from the enzyme derived from KNK269, 25 mM glyoxylic acid was generated from the enzyme derived from KNK011, 5.7 mM glyoxylic acid was generated from the enzyme derived from KNK058, and 26 mM glyoxylic acid was generated from the enzyme derived from IFO 15516.

### (Example 12)

The activity of aldehyde oxidase contained in the cultured cells of *Cellulomonas turbata* IFO 15012, *Cellulomonas turbata* IFO15014, *Cellulosimicrobium cellulans* IFO 15013, *Cellulosimicrobium cellulans* IFO 15516, *Cellulosimicrobium cellulans* JCM 6201, and *Microbacterium sp.* KNK011, was measured by the following method, respectively.

5 ml of medium (pH 7) with the composition consisting of 2 g of ammonium nitrate, 1 g of dipotassium hydrogen phosphate, 1. 3 g of sodium dihydrogen phosphate, 5 g of yeast extract, 0.2 g of magnesium sulfate heptahydrate, and 0.1 g of calcium chloride (all of which were amounts contained in 1 liter), was poured into a large test tube, and it was then sterilized by autoclaving. The aforementioned strain was inoculated into this medium using an inoculating loop, and preculture was then carried out at 28°C for 2 days. Subsequently, 1 ml of the obtained preculture broth was inoculated into 60 ml of the same above medium placed in a 500-ml Sakaguchi flask, and it was then cultured at 28°C for 2 days. Thereafter, the cells were collected from the obtained culture broth by centrifugation, and they were then washed with 100 mM potassium phosphate buffer (pH 7) twice, and then suspended in 5.0 ml of the same above buffer. 0.45 ml of a 133 mM glyoxal aqueous solution and 0.05 ml of a 50,000 U/ml catalase solution were added to 1.0 ml of the present cell suspension, and the obtained mixture was subjected to a shake reaction in a test tube at 28°C for 4 hours. Thereafter, the obtained reaction solution was analyzed by high performance liquid chromatography. As a result, it was found that glyoxylic acid was generated from glyoxal in all types of the strains.

Moreover, 0.1 ml of the cell suspension was added to 0.1 ml of a 100 mM potassium phosphate buffer comprising 50 mM glyoxal, 1.34 mM 4-AA, 2.18 mM TOOS, and 4 U/ml peroxidase, and the mixture was then shaken at 28°C for 2 hours. Thereafter, oxidase activity was evaluated by visual observation of the coloration of the reaction solution. The same above reaction solution without glyoxal was simultaneously examined as a control test. As a result, in all types of the strains, only when glyoxal was added, coloration due to oxidase activity was observed. Thus, it became clear that glyoxal was oxidized by oxidase. These results are summarized in Table 6.

**Table 6**

| Strain | Amount of glyoxylic acid produced (mM) | Coloration of reaction solution | |
|---|---|---|---|
| | | Glyoxal added | Glyoxal not added |
| *Cellulomonas turbata* IFO 15012 | 5.1 | +++++ | - |
| *Cellulosimicrobium cellulans* IFO 15013 | 8.2 | ++++ | - |
| *Cellulomonas turbata* IFO 15014 | 4.1 | +++ | - |
| *Cellulosimicrobium cellulans* IFO 15516 | 22.6 | ++++ | - |
| *Cellulosimicrobium cellulans* JCM 6201 | 20.1 | ++++ | - |
| *Microbacterium sp.* KNK011 | 4.0 | +++ | - |

### (Example 13)

Using the supernatants of the culture broths of *Microbacterium sp.* KNK011 and *Cellulosimicrobium cellulans* IFO 15516 prepared in Example 12, the secretion of aldehyde oxidase to outside of the cells thereof was confirmed. The culture broth was centrifuged, and the culture supernatant, from which cells had been removed, was concentrated by ultrafiltration using Amicon Centriplus YM-10 (manufactured by MILLIPORE). The buffer was exchanged with a 100 mM potassium phosphate buffer (pH 7), so as to prepare a 12-times concentrate. Using 0.1 ml of the thus obtained culture supernatant concentrate, oxidase activity was evaluated by coloration using glyoxal as a substrate in the same manner as in Example 12. As a result, as shown in Table 7, the culture supernatant also exhibited enzyme activity that was equivalent to that of the cell suspension.

**Table 7**

| Strain | Coloration of reaction solution | |
|---|---|---|
| | Cell suspension | Concentrated culture supernatant |
| *Cellulosimicrobium cellulans* IFO 15013 | ++++ | ++++ |
| *Microbacterium sp.* KNK011 | +++ | +++ |

### (Example 14)

The amino acid sequence of aldehyde oxidase purified from *Streptomyces sp.* KNK269 in Example 4 was determined by the following method. Reverse phase HPLC was applied to separate three types of subunits that constitute the enzyme. As a column, YMC-Pack PROTEIN-RP column (250 x 4.6 mm) (manufactured by YMC) was used. As a mobile phase, 0.1% trifluoroacetic acid was used. The subunits were separated by a method of charging 300 µg of the purified enzyme to the column and eluting it with a linear concentration gradient of acetonitrile from 0% to 56% (flow rate: 1 ml/min.; 110 minutes). A unit with a molecular weight of 25,000 was eluted at a retention time of approximately 85 minutes (hereinafter referred to as subunit γ). Another unit with a molecular weight of 35,000 was eluted at a retention time of approximately 90 minutes (hereinafter referred to as subunit β). Another unit with a molecular weight of 80,000 was eluted at a retention time of approximately 92 minutes (hereinafter referred to as subunit α). Using one-tenth of each of the separated subunits, the N-terminal amino acid sequence thereof was determined by the Edman degradation method using Protein Sequencing System 490 Procise (manufactured by Applied Biosystems). Subsequently, the internal amino acid sequence of each subunit protein was determined. The residual amount of each subunit protein as a whole was denatured with 9M urea. The buffer was exchanged with a 0.3 M Tris-HCl buffer (pH 9.0), and it was then digested with lysyl endopeptidase at 30°C for 19 hours. The degradation products were purified by the reverse phase HPLC method using an YMC-Pack PROTEIN-RP column. 0.1% trifluoroacetic acid was used as a mobile phase, and peptides were eluted with a linear concentration gradient of acetonitrile from 10% to 48%. Each peak eluted was separated, and the amino acid sequence of the inside of the subunit was determined by the same method. Of the obtained amino acid sequences, representative examples are represented by SEQ ID NOS: 15 to 20.

### (Example 15)

Based on the obtained partial amino acid sequences, mixed DNA primers (SEQ ID NOS: 21 to 26) were synthesized. Using the genomic DNA of *Streptomyces sp.* KNK269 as a template, PCR was carried out with the above mixed DNA primers in a GC buffer (manufactured by Takara Shuzo Co., Ltd.), with TAKARA LA Taq DNA polymerase (manufactured by Takara Shuzo Co., Ltd.). The obtained amplified DNA was subjected to agarose gel electrophoresis, and DNA that formed a band was extracted using QIAquick Gel Extraction kit (manufactured by QIAGEN). The obtained DNA was directly sequenced. Otherwise, it was TA cloned into pT7Blue-2 (manufactured by Novagen), and DNA sequencing was then carried out using a plasmid, so as to determine a nucleotide sequence. PCR was carried out using the primers represented by SEQ ID NOS: 21 to 26 in the combination with (1) and (2), (3) and (4), and (5) and (6), so as to obtain amplified DNA sequences. The thus obtained sequences were converted into amino acids, and these amino acids were checked against the amino acid sequences determined in Example 14. They were then aligned. As a result, it was found that genes encoding the three types of subunits were adjacent to one another on the genome in the order of γ, α, and β from the upstream, or a portion thereof overlapped. The nucleotide sequences of the mixed primer portions were determined by the same above method using peripheral nucleotide sequences as primers. Thus, the total nucleotide sequences of genomic regions encoding all the genes of the subunits γ, α, and β were determined, except for the sequence around the N-terminus of the subunit γ and the sequence around the C-terminus of the subunit α. The amino acid sequences obtained from the obtained nucleotide sequences completely matched with the partial amino acid sequences of the subunits obtained in Example 14. The determined amino acid sequences of the subunits γ, α, and β are represented by SEQ ID NOS: 1 to 3. The nucleotide sequences thereof are represented by SEQ ID NOS: 4 to 6. SEQ ID NOS: 1 and 4 indicate an amino acid sequence from the N-terminus of the purified protein of the subunit γ and a nucleotide sequence from Glu12 to the termination codon thereof, respectively. SEQ ID NOS: 2 and 5 indicate the entire amino acid sequence of the subunit β and the entire nucleotide sequence from the initiation codon to the termination codon thereof, respectively. SEQ ID NOS: 3 and 6 indicate an amino acid sequence from Met 1 to Thr 693 of the subunit α and a nucleotide sequence from the initiation codon to Arg 685 thereof, respectively. The N-terminal amino acid sequence of the subunit α of the enzyme purified from KNK269 represented by SEQ ID NO: 18 started with Ala 5 of the amino acid sequence represented by SEQ ID NO: 3 that was determined from the gene arrangement.

### (Example 16)

The amino acid sequence of aldehyde oxidase purified from *Microbacterium sp.* KNK011 in Example 5 was determined. Using 10 µg of the purified enzyme that had been desalted with an ultrafilter membrane, the N-terminal amino acid sequence thereof was determined by the same method as applied in Example 14. Subsequently, in order to determine the amino acid sequence of the inside of the protein, the amino acid sequence of the protease-digested peptide of the purified enzyme was determined. 100 µg of the purified enzyme was denatured with 9 M urea. The buffer was exchanged with a 0.2 M Tris-HCl buffer (pH 9.0), and it was then digested with lysyl endopeptidase at 30°C for 16 hours. The thus obtained digested peptide mixture was separated by the reverse phase HPLC method using an YMC-Pack PROTEIN-RP column (250 x 4.6 mm) by the same method as in Example 14. Each peak eluted was separated, and the amino acid sequence was determined by the same method, so as to determine the partial amino acid sequence of the inside of the enzyme protein. Of the obtained amino acid sequences, representative examples are represented by SEQ ID NOS: 27 to 29.

### (Example 17)

Based on the determined partial amino acids, mixed DNA primers (SEQ ID NOS: 30 to 33) were synthesized. The genomic DNA of *Microbacterium sp*. KNK011 was used as a template, and PCR was carried out by the same method as in Example 15. The amplified DNA was subjected to agarose gel electrophoresis, and DNA that formed a band was extracted using QIAquick Gel Extraction kit (manufactured by QIAGEN). The obtained DNA was directly sequenced. Otherwise, it was TA cloned into pT7Blue-2, and DNA sequencing was then carried out, so as to determine a nucleotide sequence. PCR was carried out using the primers represented by SEQ ID NOS: 30 to 33 in the combination with (1) and (2), and (3) and (4). The thus obtained nucleotide sequences were checked against the amino acid sequences. The approximately 2.8-kb nucleotide sequence of the purified enzyme was determined by the same method as in Example 15, except for the sequences around the N- and C-termini thereof. The nucleotide sequences around the N- and C-termini of the purified enzyme were determined by the inverse PCR method (refer to Cell Science, 1990, Vol. 6, No. 5, 370-376). The genomic DNA was treated with various types of restriction enzymes, and self-ligation was then carried out at a final concentration of 2.5 ng/ml. Using primers synthesized from the nucleotide sequences around the above termini, PCR was carried out. The obtained amplified DNA was ligated to pT7Blue-2, and the nucleotide sequence thereof was determined in the same manner. A sequence from upsteam of the initiation codon to downstream of the N-terminus of the purified enzyme was determined from an amplified band consisting of approximately 650 nucleotides that had been obtained by inverse PCR using the PvuI digest of the genomic DNA. On the other hand, a nucleotide sequence around the termination codon was determined from an amplified band consisting of approximately 1. 9-kb nucleotides that had been obtained by inverse PCR using the ApaI digest of the genomic DNA. The thus determined full-length gene from the initiation codon to the termination codon consisted of 3, 348 nucleotides, and also consisted of 1,115 residues in terms of amino acids. An amino acid sequence obtained from the above nucleotide sequence completely matched with the amino acid sequence obtained by the amino acid sequence analysis of the purified enzyme. The N-terminus of the enzyme purified from the cells was Val at position 47 from the initiation codon Met 1. Since *Microbacterium sp.* KNK011 produced the same enzyme in the supernatant of the culture broth thereof, it was thereby found that a portion from Met 1 to Ala 46 is cleaved as a secretory signal sequence during the secretion process. The determined entire amino acid sequence of the enzyme protein and the determined entire nucleotide sequence of the enzyme gene are represented by SEQ ID NOS: 7 and 9, respectively. The entire amino acid sequence of the mature enzyme after secretion and the nucleotide sequence encoding the above enzyme are represented by SEQ ID NOS: 8 and 10, respectively.

### (Example 18)

The gene of aldehyde oxidase obtained from *Microbacterium sp.* KNK011 that had been sequenced in Example 17 was cloned into an expression vector, and an expression experiment was then carried out. The codon of Ala 46 was substituted with an initiation codon atg. A synthesis primer to which a restriction enzyme NdeI recognition sequence had been added (SEQ ID NO: 34), and a synthesis primer of a complementary sequence wherein a restriction enzyme *Eco*RI recognition sequence had been added to 63 to 68 nucleotides downstream of the termination codon (SEQ ID NO: 35), were used. Using the genomic DNA of *Microbacterium sp.* KNK011 as a template, PCR was carried out with the above primers, so as to obtain an amplified band with approximately 3.7 kb. DNA that formed this band was subjected to agarose gel electrophoresis, and then extracted with QIAquick Gel Extraction kit (manufactured by QIAGEN), followed by digestion with *Nde*I and *Eco*RI. The digested DNA was subj ected to agarose gel electrophoresis, and extraction was then carried out in the same above manner. The obtained extract was ligated to an expression vector pUCNT (Japanese Patent Laid-Open No. 2003-116552) that had been digested with *Nde*I and *Eco*RI. Thereafter, *E. coli* DH5α was transformed with the resultant product. The obtained transformant was cultured overnight in an LB medium containing 100 µg of ampicillin. Thereafter, the culture was transferred to a fresh medium of the same type, and then cultured. Two hours later, 1 mM IPTG was added thereto, and the culture was carried out for 5 hours. Thereafter, the cultured cells were treated with SDS, and the entire protein was then subjected to SDS-polyacrylamide gel electrophoresis. As a result, a band of enzyme protein was confirmed at a position of approximately 11 kDa.

### (Example 19)

The amino acid sequence of aldehyde oxidase purified from *Cellulosimicrobium cellulans* IFO 15516 in Example 7 was determined. Using 10 µg of the purified enzyme that had been desalted with an ultrafilter membrane, the amino acid sequence thereof was determined using Protein Sequencing System Model 490 Procise (manufactured by Applied Biosystems). Subsequently, 100 µg of the purified enzyme was denatured with 9 M urea. The buffer was exchanged with a 0.2 M Tris-HCl buffer (pH 9.0), and it was then digested with lysyl endopeptidase at 30°C for 16 hours. The lysate was purified by the reverse phase HPLC method using an YMC-Pack PROTEIN-RP column (manufactured by YMC). As a mobile phase, 0.1% trifluoroacetic acid was used, and peptides were eluted with a linear concentration gradient of acetonitrile from 10% to 55%. Each peak eluted was separated, and the amino acid sequence of the inside of the protein was determined in the same manner. Of the obtained amino acid sequences, representative examples are represented by SEQ ID NOS: 36 to 38.

### (Example 20)

Mixed DNA primers (SEQ ID NOS: 39 to 41) synthesized based on the partial amino acids determined in Example 19, and a mixed DNA primer (SEQ ID NO: 42) of the complementary strand DNA consisting of 2521 to 2545 nucleotides of the aldehyde oxidase gene of *Microbacterium sp.* KNK011, were used. Using the genomic DNA of *Cellulosimicrobium cellulans* IFO 15516 as a template, PCR was carried out with the above primers. The amplified DNA obtained by PCR using the primers represented by SEQ ID NOS: 39 to 42 in combination with (1) and (2), and (3) and (4), was subjected to agarose gel electrophoresis, and DNA that formed a band was extracted using QIAquick Gel Extraction kit (manufactured by QIAGEN). The obtained DNA was directly sequenced. Otherwise, it was TA cloned into pT7Blue-2, and thereafter, DNA sequencing was carried out, so as to determine a nucleotide sequence. The thus obtained nucleotide sequence was checked against the amino acid sequence. The approximately 2.5-kb nucleotide sequence of the purified enzyme was determined by the same method as in Example 15, except for the sequences around the N- and C-termini thereof. The nucleotide sequences around the N- and C-termini of the purified enzyme were determined by the same inverse PCR method as in Example 17. The genomic DNA was treated with various types of restriction enzymes, and self-ligation was then carried out at a final concentration of 2.5 ng/ml. Using primers synthesized from the nucleotide sequences around the above termini, PCR was carried out. The obtained amplified DNA was ligated to pT7Blue-2, and the nucleotide sequence thereof was determined in the same manner. A sequence from upsteam of the initiation codon to downstream of the N-terminus of the purified enzyme was determined from an amplified band with approximately 1 kb that had been obtained by inverse PCR using the NaeI digest of the genomic DNA. On the other hand, a nucleotide sequence around the termination codon was determined from an approximately 1.1-kb amplified band that had been obtained by inverse PCR using the NcoI digest of the genomic DNA. The thus determined full-length gene from the initiation codon to the termination codon consisted of 3,324 nucleotides, and also consisted of 1,107 residues in terms of amino acids. An amino acid sequence obtained from the above nucleotide sequence completely matched with the amino acid sequence obtained by the amino acid sequence analysis of the purified enzyme. The N-terminus of the enzyme purified from the cells was Asp at position 39 from the initiation codon Met 1. In the case of enzyme secreted into the medium, a portion from Met 1 to Ala 38 was cleaved as a secretory signal sequence during the secretion process. The determined entire amino acid sequence of the enzyme protein and the determined entire nucleotide sequence of the enzyme gene are represented by SEQ ID NOS: 11 and 13, respectively. The entire amino acid sequence of the mature enzyme after secretion and the nucleotide sequence encoding the above enzyme are represented by SEQ ID NOS: 12 and 14, respectively.

### Industrial Applicability

The process for production of glyoxylic acid from glyoxal using microorganisms or enzymes of the present invention enables production of glyoxylic acid under moderate conditions. It enables production of glyoxylic acid without generation of a large amount of salts, which has been problematic in the conventional chemical synthesis methods such as the nitric acid oxidation method.

### Sequence Listing Free Text

SEQ ID NO: 1 Amino acid sequence of subunit γ of aldehyde oxidase
SEQ ID NO: 2 Amino acid sequence of subunit β of aldehyde oxidase
SEQ ID NO: 3 Amino acid sequence of subunit α of aldehyde oxidase
SEQ ID NO: 4 DNA sequence of subunit γ of aldehyde oxidase
SEQ ID NO: 5 DNA sequence of subunit β of aldehyde oxidase
SEQ ID NO: 6 DNA sequence of subunit α of aldehyde oxidase
SEQ ID NO: 7 Amino acid sequence of aldehyde oxidase containing signal peptide
SEQ ID NO: 8 Amino acid sequence of aldehyde oxidase
SEQ ID NO: 9 DNA sequence of aldehyde oxidase containing signal peptide
SEQ ID NO: 10 DNA sequence of aldehyde oxidase
SEQ ID NO: 11 Amino acid sequence of aldehyde oxidase containing signal peptide
SEQ ID NO: 12 Amino acid sequence of aldehyde oxidase
SEQ ID NO: 13 DNA sequence of aldehyde oxidase containing signal peptide
SEQ ID NO: 14 DNA sequence of aldehyde oxidase
SEQ ID NO: 15 N-terminal amino acid sequence of subunit γ of aldehyde oxidase
SEQ ID NO: 16 N-terminal amino acid sequence of subunit β of aldehyde oxidase
SEQ ID NO: 17 Amino acid sequence of subunit β of aldehyde oxidase (Ala 231 to Ala 266)
SEQ ID NO: 18 N-terminal amino acid sequence of subunit α of aldehyde oxidase
SEQ ID NO: 19 Amino acid sequence of subunit α of aldehyde oxidase (Leu 261 to Glu 298)
SEQ ID NO: 20 Amino acid sequence of subunit α of aldehyde oxidase (Gly 659 to Thr 693)
SEQ ID NO: 21 Mixed DNA primer (1) corresponding to N-terminal amino acid sequence of subunit γ of aldehyde oxidase derived from *Streptomyces sp.* KNK269
SEQ ID NO: 22 Complementary mixed DNA primer (2) corresponding to amino acid sequence (Asp 251 to Ala 258) of subunit β of aldehyde oxidase derived from *Streptomyces sp.* KNK269
SEQ ID NO: 23 Mixed DNA primer (3) corresponding to amino acid sequence (Asp 251 to Ala 258) of subunit β of aldehyde oxidase derived from *Streptomyces sp.* KNK269
SEQ ID NO: 24 Complementary mixed DNA primer (4) corresponding to amino acid sequence (Leu 274 to Glu 281) of subunit α of aldehyde oxidase derived from *Streptomyces sp.* KNK269
SEQ ID NO: 25 Mixed DNA primer (5) corresponding to amino acid sequence (Leu 274 to Glu 281) of subunit α of aldehyde oxidase derived from *Streptomyces sp.* KNK269
SEQ ID NO: 26 Complementary mixed DNA primer (6) corresponding to amino acid sequence (Leu 686 to Glu 693) of subunit α of aldehyde oxidase derived from *Streptomyces sp.* KNK269
SEQ ID NO: 27 N-terminal amino acid sequence of aldehyde oxidase
SEQ ID NO: 28 Internal amino acid sequence of aldehyde oxidase
SEQ ID NO: 29 Internal amino acid sequence of aldehyde oxidase
SEQ ID NO: 30 Mixed DNA primer (1) corresponding to N-terminal amino acid sequence of aldehyde oxidase derived from *Microbacterium sp.* KNK011
SEQ ID NO: 31 Complementary mixed DNA primer (2) corresponding to amino acid sequence (Asp 513 to Phe 521) of aldehyde oxidase derived from *Microbacterium sp.* KNK011
SEQ ID NO: 32 Mixed DNA primer (3) corresponding to amino acid sequence (Asp 513 to Phe 521) of aldehyde oxidase derived from *Microbacterium sp.* KNK011
SEQ ID NO: 33 Complementary mixed DNA primer (4) corresponding to amino acid sequence (Phe 959 to Thr 969) of aldehyde oxidase derived from *Microbacterium sp.* KNK011
SEQ ID NO: 34 DNA primer containing NdeI restriction site, which is used for cloning of aldehyde oxidase derived from *Microbacterium sp.* KNK011
SEQ ID NO: 35 DNA primer containing EcoRI restriction site, which is used for cloning of aldehyde oxidase derived from *Microbacterium sp.* KNK011
SEQ ID NO: 36 N-terminal amino acid sequence of aldehyde oxidase
SEQ ID NO: 37 Internal amino acid sequence of aldehyde oxidase
SEQ ID NO: 38 Internal amino acid sequence of aldehyde oxidase
SEQ ID NO: 39 Mixed DNA primer (1) corresponding to N-terminal amino acid sequence of aldehyde oxidase derived from *Cellulosimicrobium cellulans* IFO 15516
SEQ ID NO: 40 Complementary mixed DNA primer (2) corresponding to amino acid sequence (Ile 350 to Val 358) of aldehyde oxidase derived from *Cellulosimicrobium cellulans* IFO 15516
SEQ ID NO: 41 Mixed DNA primer (3) corresponding to amino acid sequence (Thr 176 to Thr 183) of aldehyde oxidase derived from *Cellulosimicrobium cellulans* IFO 15516
SEQ ID NO: 42 Complementary mixed DNA primer (4) corresponding to DNA sequence (2521 to 2545) of aldehyde oxidase derived from *Microbacterium sp.* KNK011

## Claims

1. A process for production of glyoxylic acid, which comprises allowing oxidoreductase that can convert glyoxal into glyoxylic acid, or at least one or a mixture consisting of two or more selected from the group consisting of a culture broth, a supernatant of the culture broth, cells, and processed products of microorganism that can produce the oxidoreductase, to act on glyoxal, so as to convert glyoxal into glyoxylic acid.

2. The process for production of glyoxylic acid according to claim 1, wherein the oxidoreductase is oxidase.

3. The process for production of glyoxylic acid according to claim 1 or 2, wherein the oxidoreductase is obtained from at least one microorganism selected from the group consisting of the genus Stenotrophomonas, Streptomyces, Pseudomonas, Microbacterium, Achromobacter, Cellulomonas, Cellulosimicrobium, and Morganella.

4. The process for production of glyoxylic acid according to claim 3, wherein the microorganism is *Stenotrophomonas sp.* KNK235 (FERM P-19002), *Streptomyces sp.* KNK269 (FERM BP-08556), *Pseudomonas sp*. KNK058 (FERM BP-08555), *Pseudomonas sp.* KNK254 (FERM P-19003), *Microbacterium sp.* KNK011 (FERM BP-08554), *Achromobacter sp*. IFO 13495, *Cellulomonas sp.* JCM 2471, *Cellulomonas turbata* IFO 15012, *Cellulomonas turbata* IFO 15014, *Cellulomonas turbata* IFO 15015, *Cellulosimicrobium cellulans* IFO 15013, *Cellulosimicrobium* *cellulans* IFO 15516, *Cellulosimicrobium cellulans* JCM 6201, or *Morganella morganii* IFO 3848.

5. The process for production of glyoxylic acid according to claim 1, wherein catalase is allowed to coexist during the reaction.

6. An aldehyde oxidase derived from a microorganism that acts on glyoxal to generate glyoxylic acid.

7. The aldehyde oxidase according to claim 6, whose activity to glyoxylic acid is one-tenth or less of its activity to glyoxal.

8. The aldehyde oxidase according to claim 6 or 7, wherein the aldehyde oxidase is produced by at least one microorganism selected from the group consisting of the genus Stenotrophomonas, Streptomyces, Pseudomonas, Microbacterium, Achromobacter, Cellulomonas, Cellulosimicrobium, and Morganella.

9. The aldehyde oxidase according to claim 8, wherein the microorganism is *Stenotrophomonas sp.* KNK235 (FERM P-19002), *Streptomyces sp.* KNK269 (FERM BP-08556), *Pseudomonas sp.* KNK058 (FERM BP-08555), *Pseudomonas sp.* KNK254 (FERM P-19003), *Microbacterium sp.* KNK011 (FERM BP-08554), *Achromobacter sp.* IFO 13495, *Cellulomonas sp.* JCM 2471, *Cellulomonas turbata* IFO 15012, *Cellulomonas turbata* IFO 15014, *Cellulomonas turbata* IFO 15015, *Cellulosimicrobium cellulans* IFO 15013, *Cellulosimicrobium cellulans* IFO 15516, *Cellulosimicrobium cellulans* JCM 6201, or *Morganella morganii* IFO 3848.

10. The aldehyde oxidase according to claim 8, which is produced by a microorganism belonging to the genus Streptomyces and which has the following physicochemical properties (1) to (3):
(1) optimum pH: 6 to 9;
(2) heat stability: the aldehyde oxidase retains activity of 90% or more after it has been treated at pH 7.2 at 60°C for 20 minutes; and
(3) molecular weight: the aldehyde oxidase has a molecular weight of approximately 110,000 in gel filtration analysis, and has three subunit proteins with molecular weights of approximately 25,000, approximately 35,000, and approximately 80,000 in SDS-polyacrylamide gel electrophoresis analysis.

11. The aldehyde oxidase according to claim 8, which is produced by a microorganism belonging to the genus Pseudomonas and which has the following physicochemical properties (1) to (3):
(1) molecular weight: approximately 150,000 in gel filtration analysis;
(2) optimum reaction temperature: 60°C to 70°C; and
(3) optimum reaction pH: 5 to 7.

12. The aldehyde oxidase according to claim 8, which is generated by a microorganism belonging to the genus Microbacterium inside and outside of the cells thereof and which has the following physicochemical properties:
molecular weight: a single protein has a molecular weight of approximately 110,000 in SDS-polyacrylamide gel electrophoresis analysis.

13. The aldehyde oxidase according to claim 8, which is generated by a microorganism belonging to the genus Cellulosimicrobium inside and outside of the cells thereof, and which has the following physicochemical properties:
molecular weight: a single protein has a molecular weight of approximately 90,000 to 100,000 in SDS-polyacrylamide gel electrophoresis analysis.

14. The aldehyde oxidase according to claim 10, wherein the microorganism belonging to the genus Streptomyces is *Streptomyces sp.* KNK269 (FERM BP-08556).

15. The aldehyde oxidase according to claim 11, wherein the microorganism belonging to the genus Pseudomonas is *Pseudomonas sp.* KNK058 (FERM BP-08555).

16. The aldehyde oxidase according to claim 12, wherein the microorganism belonging to the genus Microbacterium is *Microbacterium sp.* KNK011 (FERM BP-08554).

17. The aldehyde oxidase according to claim 13, wherein the microorganism belonging to the genus Cellulosimicrobium is *Cellulosimicrobium cellulans* IFO 15516.

18. The aldehyde oxidase according to claim 6, which has a protein described in the following (a) or (b) as a subunit:
(a) a protein having an amino acid sequence represented by SEQ ID NO: 1, 2, or 3; or
(b) a protein comprising an amino acid sequence result from deletion, substitution, or addition of one or several amino acids in the amino acid sequence (a).

19. The aldehyde oxidase according to claim 6, which has a protein encoded by the DNA described in the following (a) or (b) as a subunit:
(a) DNA having a nucleotide sequence represented by SEQ ID NO: 4, 5, or 6; or
(b) DNA which hybridizes with any one DNA consisting of a nucleotide sequence that is complementary to the DNA consisting of the nucleotide sequence (a) under stringent conditions.

20. The aldehyde oxidase according to claim 6, which has the amino acid sequence described in the following (a) or (b):
(a) an amino acid sequence represented by SEQ ID NO: 7, 8, 11, or 12; or
(b) an amino acid sequence resulting from deletion, substitution, or addition of one or several amino acids in the amino acid sequence (a).

21. The aldehyde oxidase according to claim 6, which is encoded by the DNA described in the following (a) or (b):
(a) DNA having a nucleotide sequence represented by SEQ ID NO: 9, 10, 13, or 14; or
(b) DNA which hybridizes with DNA consisting of a nucleotide sequence that is complementary to the DNA consisting of the nucleotide sequence (a) under stringent conditions.

22. DNA encoding a subunit of the aldehyde oxidase according to claim 6, which comprises the DNA described in the following (a) or (b):
(a) DNA having a nucleotide sequence represented by SEQ ID NO: 4, 5, or 6; or
(b) DNA which hybridizes with any one DNA consisting of a nucleotide sequence that is complementary to the DNA consisting of the nucleotide sequence (a) under stringent conditions.

23. DNA encoding the aldehyde oxidase according to claim 6, which comprises the DNA described in the following (a) or (b) :
(a) DNA having a nucleotide sequence represented by SEQ ID NO: 9, 10, 13, or 14; or
(b) DNA which hybridizes with any one DNA consisting of a nucleotide sequence that is complementary to the DNA having the nucleotide sequence (a) under stringent conditions.

24. DNA encoding a subunit of the aldehyde oxidase according to claim 6, which comprises an amino acid sequence resulting from deletion, substitution, or addition of one or several amino acids in the amino acid sequence represented by SEQ ID NO: 1, 2, or 3.

25. DNA encoding the aldehyde oxidase according to claim 6, which comprises an amino acid sequence resulting from deletion, substitution, or addition of one or several amino acids in the amino acid sequence represented by SEQ ID NO: 7, 8, 11, or 12.
